(19)

**Europäisches Patentamt**

**European Patent Office**

**Office européen des brevets**

(11) **EP 4 674 857 A2**

(12) **EUROPEAN PATENT APPLICATION**

(43) Date of publication:
**07.01.2026 Bulletin 2026/02**

(21) Application number: **25201703.3**

(22) Date of filing: **19.02.2020**

(51) International Patent Classification (IPC):
*C07F 7/08* (2006.01)

(52) Cooperative Patent Classification (CPC):
**A61K 8/11; A61K 8/025; A61K 8/35; A61K 8/361; A61K 8/894; A61Q 19/00;** A61K 9/5115; A61K 2800/10; A61K 2800/412

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.02.2019 US 201962808481 P**

(62) Document number(s) of the earlier application(s) in accordance with Art. 76 EPC:
**20759022.5 / 3 927 379**

(71) Applicant: **Pharma in silica Laboratories inc.**
**Québec QC G1P 4S6 (CA)**

(72) Inventors:
• **BOUCHOUCHA, Meryem**
**Québec G1J 4M1 (CA)**
• **WU, Xiaowei**
**Québec G1P 4S6 (CA)**
• **GIRET, Simon**
**Québec G1N 1C2 (CA)**

• **DESPLANTIER-GISCARD, Delphine**
**Québec G0Q 2R0 (CA)**
• **ABOSHYAN-SORGHO, Lilit**
**Québec G1V 3X9 (CA)**
• **PANDARUS, Valerica**
**Québec G1Y 3L3 (CA)**
• **MORIN, Michel**
**Saint-Augustin-de-Desmaures G3A 3B9 (CA)**

(74) Representative: **Regimbeau**
**20, rue de Chazelles**
**75847 Paris Cedex 17 (FR)**

Remarks:
•This application was filed on 11-09-2025 as a divisional application to the application mentioned under INID code 62.
•Claims filed after the date of filing of the application / after the date of receipt of the divisional application (Rule 68(4) EPC).

(54) **DIRECT NANOEMULSION PROCESS FOR THE SYNTHESIS OF SPHEROIDAL ORGANOSILOXANE SUB-MICRON/NANOPARTICLES**

(57) The present disclosure relates to spheroidal organosiloxane submicron/nanoparticle comprising a network consisting of organosiloxane, and a process to make them.

EP 4 674 857 A2

Description

FIELD OF THE DISCLOSURE

The present disclosure relates to spheroidal organosiloxane sub-micron/nanoparticles comprising a network consisting of organosiloxane, and a process to make them.

BACKGROUND

Amorphous silica sub-micron/nanoparticles are considered as very attractive advanced materials for numerous applications in catalysis, analytical extraction, sensing, optics, cosmetics, pharmaceutics or additives industries. These silica sub-micron/nanoparticles present a high surface area, suitable cost, versatile compositions, thermic/chemical stability, inertness and desirable innocuousness. What's more, silica is Generally Recognized As Safe (GRAS) by the Food and Drug Administration (FDA), and silica sub-micron/nanoparticles can be synthesized with or without actives/payloads to meet the demands of the applications.
Since 1970, the domains of drug delivery systems and controlled release have undergone an impressive progress. Silica sub-micron/nanoparticles have emerged as one of the most promising materials for therapeutic, diagnostic or theragnostic applications, owing to their high potential as a controlled release drug carrier or bioimaging probes support. However, it is still a great challenge to achieve the desired physicochemical properties, particularly in the field of pharmaceutics.
A strategy, which allows the simple adjustment of physicochemistry properties of sub-micron/nanoparticles and reduces the synthesis steps, will be considered.

SUMMARY OF THE DISCLOSURE

In one aspect, there is provided a process of preparation of spheroidal organosiloxane submicron/nanoparticles comprising:

i1) separately hydrolyzing at least one organosiloxane precursor in a hydrolytic media to provide one or more pre-hydrolyzed organosiloxane precursor;

i2) combining the pre-hydrolyzed organosiloxane precursors of step i1) to provide a combined pre-hydrolyzed organosiloxane precursor;

i3) removing a part or totality of volatile solvents from said combined pre-hydrolyzed organosiloxane precursors to provide a dispersed phase comprising pre-condensed organosiloxane precursors;

i4) emulsifying, in absence of a surfactant and with (an adjusted) shear force or sonication, the dispersed phase of the step i3) in an aqueous continuous phase to provide an oil in water nanoemulsion; and

i5) adding a condensation catalyst to the nanoemulsion of step i4) to obtain the spheroidal organosiloxane submicron/nanoparticles suspension.

In a further aspect, there is provided a spheroidal organosiloxane sub-micron/nanoparticle comprising a network consisting of organosiloxane, wherein said spheroidal organosiloxane sub-micron/nanoparticle is uncalcined amorphous, surfactant-free and is nano to submicron size, said particle optionally comprising an active/payload.

BRIEF DESCRIPTION OF THE FIGURES

The illustrations of the examples corresponding figures are listed as bellow:

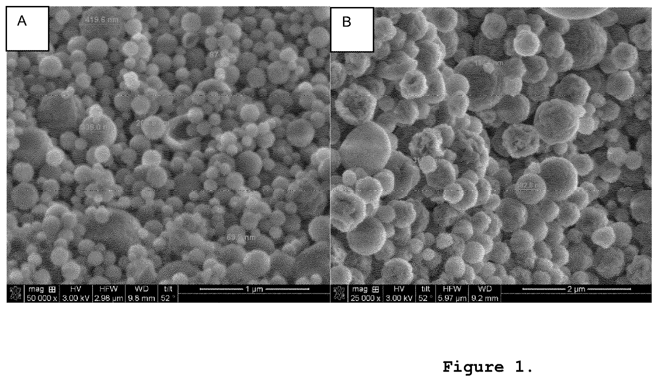

**Figure 1.** A) SEM (scanning electronic microscopy) of Example 1-1 (scale bar = 1 $\mu$m); B) SEM (scanning electronic microscopy) of Example 1-2 (scale bar = 1 $\mu$m)

**Figure 2.** A) SEM (scanning electronic microscopy) of Example 2 (scale bar = 1 $\mu$m); B) Example 3 (scale bar = 1 $\mu$m)

**Figure 3.** SEM of A) Example 4 (scale bar = 5 $\mu$m); B) Example 5 (scale bar = 5 $\mu$m)

**Figure 4.** Comparison of the particles size distribution obtained in example 4 and example 5 by DLS.

**Figure 5.** SEM of A) Example 6 (scale bar = 1 μm); B) Example 7 (scale bar = 1 μm); C) Example 8 (scale bar = 3 μm); D) Example 9 (scale bar = 1 μm)

**Figure 6.** SEM of A) Example 10 (scale bar = 3 μm); B) Example 11 (scale bar = 2 μm); C) Example 12 (scale bar = 1 μm); D) Example 13 (scale bar = 1 μm)

**Figure 7.** SEM of A) Example 14 (scale bar = 20 μm); B) Example 15(scale bar = 1 μm); C) Example 16(scale bar = 500 nm); D) Example 17 (scale bar = 1 μm); E) Example 18(scale bar = 1 μm); F) Example 19(scale bar = 3 μm); G) Example 20 (scale bar = 1 μm); H) Example 21(scale bar = 1 μm); I) Example 22(scale bar = 3 μm); J) Example 23 (scale bar = 1 μm); K) Example 24(scale bar = 1 μm); L) Example 25 (scale bar = 500 nm); M) Example 26 (scale bar = 500 nm).

**Figure 8.** SEM of Example 27 (scale bar = 4 μm) (on the left) and example 28 (scale bar = 3 μm) (on the right).

**Figure 9.** SEM of Example 29 (scale bar = 5 μm).

**Figure 10.** SEM of Example 30 (scale bar = 4 μm) (top) and Particle size distribution of the corresponding spheroidal organisiloxane sub-micron/nanoparticles (bottom).

## DETAILED DESCRIPTION

The disclosure relates to a strategy of preparing oil in water nanoemulsion, which leads to the formation of spheroidal organosiloxane sub-micron/nanoparticles in one pot as opposed to the multistep nanoparticles' synthesis. The external surface of the sub-micron/nanoparticles can be modified and payloads may be incorporated in-situ to avoid tedious and time-consuming post-grafting and post-impregnation steps.

The disclosure provides a spheroidal organosiloxane sub-micon/nanoparticles synthesis process. This process is providing: 1) formation of a direct phase nanoemulsion process (i.e. oil in water (O/W)), 2) in-situ and/or ex-situ surface functionalization process, 3) with or without in-situ actives/payloads administration method to distribute the active ingredients throughout the spheroidal organosiloxane submicron/nanoparticles, 4) choice of spheroidal organosiloxane sub-micron/nanoparticle matrices by varying the silica precursors composition, 5) preventing the undesired release or degradation of the entrapped actives, and 6) possible actives/payloads release control by tailoring the physiochemical properties of the external and internal surface of the developed spheroidal organosiloxane sub-micron/nanoparticles.

It is known to the skilled worker that the scale of "nanoparticles" is less than 100 nm and that size of "submicron" particles is between about 100 nm and 1 μm.

The process herein is preferably conducted under high shear or high dispersing force.

The process herein is conducted without surfactant.

The process herein is optionally conducted with one or more nanoemulsion stabilizer.

A surfactant or a nanoemulsion stabilizer is understood of any such agent not taking part in the siloxane network (forming Si-O-Si) bonds. Certain organosiloxane precursors used herein may have amphiphilic parts but are however not excluded from the process herein as they participate in creating the siloxane network.

The "organosiloxane precursors" used herein refer to compounds of formula $R_{4-x}Si(L)_x$ or formula $(L)_3Si\text{-}R'\text{-}Si(L)_3$, wherein;

R: is an alkyl, alkenyl, alkynyl, alicyclic, aryl, alkyl-aryl group, which is optionally substituted by a halogen atom, -OH, -SH, polyethylene glycol (PEG), $-N(R_a)_2$, $-N^+(R_a)_3$, $-P(R_a)_2$; $R_a$ can be alkyl, alkenyl, alkynyl, alicyclic, aryl and alkyl-aryl.

L: is a halogen or an acetoxide $-O\text{-}C(O)R_a$, or alkoxide $OR_a$ group.

X: is an integer of 1 to 4.

R': is an alkyl, alkenyl, alkynyl, alicyclic, aryl, alkyl-aryl group, which is optionally substituted by a halogen atom, -OH, -SH, $-N(R_a)_2$, $-N^+(R_a)_3$, $-P(R_a)_2$.

In one embodiment, the organosiloxane precursor $R_{4-x}Si(L)_x$ or $(L)_3Si\text{-}R'\text{-}Si(L)_3$ is a silicon alkoxide such as a tetraalkoxide silane, a monoalkyl-trialkoxysilane, a dialkyl dialkoxysilane or a bis- trialkoxy bridged silane. In a further aspect the organosiloxane precursor is a mixture of silicon alkoxides, such as tetraalkoxy silane and/or monoalkyl-trialkoxysilane, and/or dialkyl-dialkoxysilane and/or a bis-trialkoxy bridged silane.

In one embodiment, the monoalkyl trialkoxy silanes $RSi(L)_3$ comprise monoalkyl, which is linear or branched group of 1 to 18 carbon atoms, and the trialkoxy is triethoxy or trimethoxy group.

In one embodiment, the dialkyl dialkoxy silanes $R_2Si(L)_2$ comprise dialkyl, which is linear or branched group of 1 to 18 carbon atoms, and the dialkoxy is diethoxy or dimethoxy group.

In one embodiment, the trialkoxy bridged silanes $(L)_3Si$-R'-$Si(L)_3$ comprise bridged, which is linear alkyl or alkenyl group of 2 to 18 carbon atoms, and the trialkoxy is triethoxy or trimethoxy group.

In one embodiment, the PEG comprises linear or branched $-(OCH_2CH_2)-$ units. The PEG-silane refers to $(L)_3Si$-$(OCH_2CH_2)_n$-R. The PEG-silane molecular weight is between 400 and 20000 Da.

The "hydrolytic media" used herein refers to any chemical reagents which favor the formation of silanol function Si-OH produced from the hydrolysis of the organosiloxane precursors. Examples of such media include aqueous medias, such as water, optionally mixed with a water miscible organic solvent, such as ethanol or THF and an inorganic acid catalyst such as HCl, $H_3PO_4$, $H_2SO_4$, $HNO_3$. Preferably the inorganic acid catalyst is HCl with a concentration in the hydrolytic media from about 0.01 mol.$l^{-1}$ to 0.05 mol.$l^{-1}$.

The "nanoemulsion stabilizer" used herein refers to a carboxylic acid (COOH)- containing compound, which further stabilize the nanoemulsion, leading to better control of the particle size distribution of the obtained spheroidal organosiloxane sub-micron/nanoparticles. Preferably, the carboxylic acid containing compound is comprising an aliphatic chain ($R_b$), which is either saturated or unsaturated. $R_b$ can be alkyl, alkenyl, alkynyl with at least 8 carbon atoms.

In one embodiment, the carboxylic acid is octanoic acid or branched derivatives (e.g. 4-methyl-n-octanoic acid and 2-methylheptanoic acid). In one embodiment, the carboxylic acid is oleic acid.

The "condensation catalyst" used herein refers to any reagent known in the art to favor the polycondensation to form siloxane bonds Si-O-Si.

In one embodiment, the condensation catalyst achieves a final pH in the suspension at about 9.0 to 11.5. The condensation catalyst can be, but not limited to, $NH_4OH$, NaOH, KOH, LiOH, $Ca(OH)_2$, NaF, KF, TBAF, TBAOH, TMAOH, triethanol amine (TEA), triethyl amine, primene, L-lysine, aminopropylsilane.

In one embodiment, the condensation catalyst is concentrated $NH_4OH$. In one embodiment, the condensation catalyst is NaOH. In one embodiment, the condensation catalyst is TEA.

The "dispersed phase" used herein means the mixture of the pre-condensed organosiloxane precursors, with or without actives/payloads and with or without the nanoemulsion stabilizer. Pre-condensed organosiloxane precursors are obtained by the partial condensation of the pre-hydrolyzed organosiloxane precursors by evaporating the volatile solvents present in the hydrolytic media. Pre-hydrolyzed organosiloxane precursors are obtained by the hydrolysis of the L group of $R_{4-x}Si(Li)_x$ or $(L)_3SiR'$-$Si(L)_3$ in the hydrolytic media.

The "continuous phase" used herein means solvent known in the art to have opposite polarity compared to pre-condensed organosiloxane precursors to produce direct phase nanoemulsion (oil in water).

In one embodiment, the continuous phase is water.

In one embodiment, the weight ratio of the continuous phase to the dispersed phase containing the pre-condensed organosiloxane precursors is 25 to 500, preferably 50 to 200.

The "nanoemulsion process" used herein indicates a process that involves a piece of laboratory or industrial equipment used to mix two or more liquids that are normally immiscible. Preferably rotor-stator homogenizer, sonic dismembrator or in continuous inline method. The process results in the formation of nanodroplets of the dispersed phase in the continuous phase.

In one embodiment, rotor-stator homogenizer is used for the nanoemulsion process. Typically, the homogenizer speed is from 10000 rpm to 25000 rpm. Preferably, from 15000 rpm to 20000 rpm.

In one embodiment, sonic dismembrator homogenizer is used for the emulsion process. Typically, the homogenizer power potentiometer is about 5 % to 100 % (such as from 20 W to 750 W or 20 W to 400 W) with an on/off cycle on from 50 % to 100 % of the time. Preferably, in the range of 50-150 or about 50 W for power potentiometer and 100% on for the cycle time.

The particle size and mono-/polydispersity degree of the spheroidal organosiloxane sub-micron/nanoparticles depends on the speed of the rotor-stator homogenizer or the power of the sonic dismembrator. The higher the speed or power is, the lower the polydispersity degree of the spheroidal organosiloxane submicron/nanoparticles is. The same tendency is observed for the particles size, the higher the speed or power is, the lower the particle size is.

The "actives/payloads" used herein refer to the compounds of interests which will be trapped in the spheroidal organosiloxane sub-micron/nanoparticles. Actives/payloads are preferably insoluble in the continuous phase. The actives/payloads can be in both solid and liquid form. They can be incorporated by solubilization or dispersion into the pre-condensed organosiloxane precursors. The active can be incorporated in controlled environment (e.g. under argon for air sensitive compounds and in dark for light sensitive compounds).

This "actives/payloads" used can be liposoluble pharmaceutic or cosmetic actives, such as drugs, essential oil, fragrances, perfumes, as well as other liposoluble chemical actives.

In one embodiment, benzophenone is used as active/payload. In one embodiment, $\alpha$-pinene is used as active/payload. In one embodiment, vitamin A acetate is used as active/payload. In one embodiment, a taxane (e.g.

paclitaxel, docetaxel) is used as active/payload.

The process of preparing spheroidal organosiloxane submicron/nanoparticles comprises: 1) separately hydrolyzing at least one organosiloxane precursor in a hydrolytic media to provide one or more pre-hydrolyzed organosiloxane precursor(s). Then, combining all the pre-hydrolyzed organosiloxane precursors to provide a combined pre-hydrolyzed organosiloxane precursor of step 1); 2) removing a part or totality of the volatile solvents from said combined pre-hydrolyzed organosiloxane precursors to provide a dispersed phase comprising pre-condensed organosiloxane precursors; 3) optionally adding a nanoemulsion stabilizer in the pre-condensed organosiloxane precursors to provide the dispersed phase; 4) optionally adding an active/payload into the dispersed phase (of step 2 or Step 3); 5) emulsifying the dispersed phase (of step 2 or step 3 or step 4) in a continuous phase optionally containing a nanoemulsion stabilizer, wherein said emulsion stabilizer is the same or different from the emulsion stabilizer in said disperse phase; 6) mixing the emulsion of the step 5) with a condensation catalyst to obtain a suspension of sub-micron/nanoparticles, 7) optionally aging the suspension and 8) optionally isolating, washing and/or drying the final spheroidal organosiloxane submicron/-nanoparticles.

In one embodiment, at room temperature, in the hydrolytic media, all the organosiloxane precursors are hydrolyzed independently with vigorous agitation, for example, at the stirring rate of at least 500 rpm for minimum 1 hour, and combined into one container. (Step 1)

In one embodiment, the pre-condensed organosiloxane precursor's phase is resulted following partial or total removal of volatile solvents. (Step 2)

In one embodiment, the nanoemulsion stabilizer can be optionally mixed with the pre-condensed organosiloxane precursors at the end of step 2. (Step 3)

In one embodiment, when spheroidal organosiloxane submicron/nanoparticles contained actives/payloads, the actives/payloads, in solid or liquid state, are introduced in the resulting dispersed phase at step 2 or Step 3. (Step 4)

In one embodiment, the active/payload, in liquid or solid state, is added or solubilized in the pre-condensed organosiloxane precursors. In another embodiment, the solid state active/payload is solubilized in the nanoemulsion stabilizer. In a further embodiment, the solid state active/payload is solubilized in a co-solvent (e.g. diethylene glycol monoethylether (DGME), triacetin, 2-pentanol) and the resulting solution is mixed homogeneously with the dispersed phase. The viscosity and polarity of the resulted dispersed phase are thus adjusted. (Step 4)

In one embodiment, the nanoemulsion stabilizer is optionally mixed in the continuous phase. In the same embodiment, the emulsification of the dispersed phase in the continuous phase can be realized with a rotor-stator homogenizer, for example, for at least 1 min. In the same embodiment, the emulsification of the dispersed phase in the continuous phase can be done with a sonic dismembrator, for example, for at least 1 min. (Step 5)

In one embodiment, during the emulsification, the condensation catalyst is added to the suspension and the emulsification process is maintained, for example for at least 15 s. (Step 6)

In one embodiment, after step 3 or step 4 or step 5 or step 6, optionally the external surface can be functionalized by adding organosiloxane precursors with or without pre-hydrolyzation. In the same embodiment, the obtained suspension is optionally aged at room temperature with stirring or shaking to maintain the stable suspension and avoid aggregation for example, for 12 to 24 h. (Step 7)

In one embodiment, the spheroidal organosiloxane submicron/microparticles are optionally isolated by centrifugation at 15K G at least during 10 min. In the same embodiment, spheroidal organosiloxane sub-micron/nanoparticles are washed with water until the supernatant reaches neutrality. Finally, the resulting material is dried at room temperature or up to 70 °C depending the properties of the actives/payloads, at atmospheric pressure or under reduced pressure, for example for at least one day. (step 8)

The trapped actives/payloads quantity is determined by analytical methods, such as high-performance liquid chromatography (HPLC), elemental analysis (EA) or thermogravimetric analysis (TGA).

The sequestration yield is defined by the following formula (equation 1). The experimental active mass corresponds to the active quantified by analytical methods. The theoretical active mass corresponds to initial introduced quantity. The sequestration yield is comprised from 50 to 100%.

$$Sequestration\ yield = \frac{m_{Active\ (Experimental)}}{m_{Active\ (Theoritical)}} \times 100 \quad \text{(Equation 1)}$$

The loading capacity is defined by the following formula (equation 2). The experimental active mass corresponds to the active quantified by analytical methods. The total mass corresponds to the mass of resulting spheroidal organosiloxane sub-micron/nanoparticles, excluded water content. The loading capacity is actives/payloads-dependent. In one embodiment, the loading capacity is from 0.1 wt % to 50 wt %.

$$Loading\ capacity = \frac{m_{Active\,(Experimental)}}{m_{total}} \times 100 \quad \text{(Equation 2)}$$

In all the embodiments, the porous structures of the spheroidal organosiloxane sub-micron/nanoparticles are non-organised. The nitrogen adsorption/desorption isotherms allow to determine the BET (Brunauer-Emmett-Teller) surface area of the spheroidal organosiloxane sub-micron/nanoparticles, which is typically up to 1000 $m^2.g^{-1}$.

The monodispersity, the re-dispersibility, the absence of aggregates and the colloidal stability of the spheroidal organosiloxane sub-micron/nanoparticles depend on the choice of organosiloxane precursors and the condensation catalyst. In one embodiment, when TEA is used as condensation catalyst, the spheroidal organosiloxane sub-micron/nanoparticles reveal high monodispersity degree (i.e. narrow particle size distribution) and colloidal stability. As confirmed by dynamic light scattering analysis (DLS), the lowest polydispersity index (PDI = 0.04) and hydrodynamic diameter are obtained when TEA is used as condensation catalyst as in example 4. The obtained particle size distribution is between 70 and 200 nm (example 4). Smaller particles with higher PDI (0.2-0.3) are obtained when PEG-silane (with molecular weight of 5000 Da) or APTES are used as organosiloxane precursors. Nanoparticles between 40 and 110 nm (example 25) and between 20-150 nm (example 26) are obtained, respectively. The re-dispersibility degree of the obtained particles is between 50 and 100 %, preferentially between 80 and 100%.

Although the spheroidal organosiloxane submicron/nanoparticles tend to be hydrophilic due to oil in water nanoemulsion process. The external surface physicochemical properties (e.g. hydrophilicity, electronegativity) of the spheroidal organosiloxane sub-micron/nanoparticles can be adjusted by tuning the following parameters: a) the concoction of the organosiloxane precursors; b) the nature of actives/payloads, and c) the in-situ functionalization of external surface during the process.

In one embodiment, when only TEOS and aliphatic organosiloxane precursors are used for the synthesis of the spheroidal organosiloxane sub-micron/nanoparticles, the zeta potential analysis exhibits a strongly negative surface charge value between -15 mV and -55 mV (at pH ≈ 6). This indicates the presence of accessible deprotonated silanol groups on the external surface. In other embodiment, when APTES (aminopropyl triethoxysilane) is used as the organosiloxane precursors, a positive zeta potential is obtained (+25 mV, at pH ≈ 6), confirming therefore the presence of positively charged amine groups at the external surface. In further embodiment, when TMAPS (3-(trimethoxysilyl) propyl] ammonium chloride) is used for external surface functionalization, a positive zeta potential (+8 mV) is obtained. This confirms the presence of ammonium ions at the external surface of spheroidal organosiloxane submicron/nanoparticles and evident the accessibility of the positively charged ammonium. In other embodiment, when polyethylene glycol silane (PEG-silane) is used (i.e. PEGylation) as for external surface functionalization, an increase in zeta potential value is recorded (close to neutral), compared to the spheroidal organosiloxane submicron/nanoparticles obtained without this external functionalization step: the zeta potential of the suspension of the spheroidal organosiloxane sub-micron/nanoparticles, in phosphate buffered saline solution (PBS), increase from -47 mV to -11 mV. This suggests the presence of PEG-silane at the external surface of spheroidal organosiloxane submicron/nanoparticles. All these conclusions are confirmed by the analysis of the outer surface composition of the spheroidal organosiloxane sub-micron/nanoparticles, using X-ray photoelectron spectroscopy (XPS). Indeed, XPS data reveal the elemental composition (%Si(2s), %C(1s), %O(1s)) of the outer surface of the obtained spheroidal organosiloxane submicron/nanoparticles in the first 5 nm in depth. The results show an increase in carbon to silicon ratio (C/Si) from 1.12 to 2.36 and 1.12 to 2.03 respectively after external surface functionalization with TMAPS and PEG-silane, compared to the spheroidal organosiloxane sub-micron/nanoparticles obtained without this external functionalization step.

In one embodiment, the spheroidal organosiloxane submicron/nanoparticles are synthesized following the general procedure without active/payload and without the help of an emulsion stabilizer. The organosiloxane precursors are preferentially a combination of methyltriethoxysilane (C1-TES), octyltriethoxysilane (C8-TES) and tetraethylorthosilicate (TEOS) in the molar percent range of respectively 10%-22.5%/5%-7.5%/85%-70% and preferentially the molar percent composition of 22.5%/7.5%/70%. The pre-condensed organosiloxane precursor phase is obtained following partial or total removal of volatile solvents. The condensation catalyst is an inorganic base, preferentially concentrated $NH_4OH$. The emulsion is obtained preferentially by high force sonicator equipment at a power from 20 W to 400 W and preferentially 50 W. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and without the help of an emulsion stabilizer. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The condensation catalyst is an organic base, preferentially TEA (triethanolamine). The emulsion is obtained preferentially by high force sonicator equipment at a power from 20 W to 400 W and preferentially 50 W. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and without the help of an emulsion stabilizer. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The condensation catalyst is an inorganic base, preferentially concentrated $NH_4OH$. The emulsion is obtained preferentially by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and without the help of an emulsion stabilizer. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The condensation catalyst is an organic base, preferentially TEA. The emulsion is obtained preferentially by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 w%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an organic base, preferentially TEA. The emulsion is obtained by high force sonicator equipment at a power from 20 W to 400 W and preferentially 50 W. The resulted spheroidal organosiloxane submicron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is TEA. The emulsion is obtained by high shear mixer at preferentially speed rate from 10 000 RPM to 25 000 RPM and preferentially 18 000 RPM. The resulted spheroidal organosiloxane submicron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the 4-methyl-n-octanoic. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The 4-methyl-n-octanoic oil is added to the pre-condensed organosiloxane precursor's phase at a weight ratio from 25 wt% to 50 wt% and preferentially 35 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the 4-methyl-n-octanoic. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The 4-methyl-n-octanoic oil is added to the pre-condensed organosiloxane precursor's phase at a weight ratio from 25 wt% to 50 wt% and preferentially 35 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the 4-methyl-n-octanoic. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The 4-methyl-n-octanoic oil is added to the pre-condensed organosiloxane precursor's phase at a weight ratio from 25 wt% to 50 wt% and preferentially 35 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane submicron/nanoparticles present an average particles size between 50 nm and 800 nm.

In one embodiment, the spheroidal organosiloxane submicron/nanoparticles are synthesized following the general procedure without active/payload and with the help of an emulsion stabilizer. The emulsion stabilizer is the 4-methyl-n-octanoic. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. 4-methyl-n-octanoic oil is added to the pre-condensed organosiloxane precursor's phase at a weight ratio from 25 wt% to 50 wt% and preferentially 35 wt%. The condensation catalyst is TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload, with the help of an emulsion stabilizer and with modification of the submicron/nanoparticle's zeta potential. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high force sonicator equipment at a power from 20 W to 400 W and preferentially 50 W. After one hour of aging, non pre-hydrolyzed (trimethoxysilylpropyl-N,N,N-trimethyl ammonium chloride (TMAPS) was added to the suspension at a molar ratio TMAPS:TEOS from 0 to 20 and preferentially 9. The resulted spheroidal organosiloxane sub-micron/nanoparticles present an average particles size between 50 nm and 800 nm. The negative zeta potential usually resulted without the ammonium silane (around -50 mV) became positive, +8 mV was measured by DLS.

In one embodiment, the spheroidal organosiloxane submicron/nanoparticles are synthesized following the general procedure without active/payload, with the help of an emulsion stabilizer and with modification of the submicron/nanoparticle's zeta potential. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an organic base, preferentially TEA. The emulsion is obtained by high force sonicator equipment at a power from 20 W to 400 W and preferentially 50 W. After one hour of aging, non pre-hydrolyzed TMAPS was added to the suspension at a molar ratio TMAPS:TEOS from 0 to 20 and preferentially 9. The resulted spheroidal organosiloxane sub-micron/nanoparticles present an average particles size between 50 nm and 800 nm. The negative zeta potential usually resulted without the ammonium silane (around -50 mV) became positive, +8 mV was measured by DLS.

In one embodiment, the spheroidal organosiloxane submicron/nanoparticles are synthesized following the general procedure without active/payload, with the help of an emulsion stabilizer and with modification of the submicron/nanoparticle's zeta potential. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. After one hour of aging, non pre-hydrolyzed TMAPS was added to the suspension at a molar ratio TMAPS:TEOS from 0 to 20 and preferentially 9. The resulted spheroidal organosiloxane submicron/nanoparticles present an average particles size between 50 nm and 800 nm. The negative zeta potential usually resulted without the ammonium silane (around -50 mV) became positive, +8 mV was measured by DLS.

In one embodiment, the spheroidal organosiloxane submicron/nanoparticles are synthesized following the general procedure without active/payload, with the help of an emulsion stabilizer and with modification of the submicron/nanoparticle's zeta potential. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is TEA. The emulsion is obtained by high shear mixer at preferentially speed rate from 10 000 RPM to 25 000 RPM and preferentially 18 000 RPM. After one hour of aging, non pre-hydrolyzed TMAPS was added to the suspension at a molar ratio TMAPS:TEOS from 0 to 20 and preferentially 9. The resulted spheroidal organosiloxane sub-micron/nanoparticles present an average particles size between 50 nm and 800 nm. The negative zeta potential usually resulted without the ammonium silane (around -50 mV) became positive, +8 mV was measured by DLS.

In one embodiment, the spheroidal organosiloxane submicron/nanoparticles are synthesized following the general procedure without active/payload, with the help of an emulsion stabilizer, with PEGylation of the external surface and modification of the sub-micron/nanoparticle's zeta potential. The emulsion stabilizer is the octanoic acid. The organosiloxane precursors are preferentially a combination of TMAPS, C8-TES, 2-methoxy(polyethyleneoxy)6-9 propyl-trimethoxysilane ($PEG_{6-9}$-TMS) and TEOS in the molar percent range of respectively 10%-22.5%/5%-7.5%/0.5%-10%/85%-70% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane submicron/nanoparticles present an average particles size between 50 nm and 800 nm. The negative zeta potential increases from -50 mV (unmodified submicron/nanoparticles) to -30 mV.

In one embodiment, the spheroidal organosiloxane submicron/nanoparticles are synthesized following the general procedure without active/payload, with the help of an emulsion stabilizer, with PEGylation of the external surface and modification of the sub-micron/nanoparticle's zeta potential. The emulsion stabilizer is the octanoic acid. The organosiloxane precursors are preferentially a combination of TMAPS, C8-TES, $PEG_{6-9}$-TMS and TEOS in the molar percent range of respectively 10%-22.5%/5%-7.5%/0.5%-10%/85%-70% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size between 50 nm and 800 nm. The negative zeta potential increases from -50 mV (unmodified submicron/nanoparticles) to -30 mV.

In one embodiment, the spheroidal organosiloxane submicron/nanoparticles are synthesized following the general procedure without active/payload, with the help of an emulsion stabilizer, with PEGylation of the external surface and modification of the sub-micron/nanoparticle's zeta potential. The emulsion stabilizer is the octanoic acid. The organosiloxane precursors are preferentially a combination of TMAPS, C8-TES, $PEG_{6-9}$-TMS and TEOS in the molar percent range of respectively 10%-22.5%/5%-7.5%/0.5%-10%/85%-70% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm. The negative zeta potential increases from -50 mV (unmodified submicron/nanoparticles) to -30 mV.

In one embodiment, the spheroidal organosiloxane submicron/nanoparticles are synthesized following the general procedure without active/payload, with the help of an emulsion stabilizer, with PEGylation of the external surface and modification of the sub-micron/nanoparticle's zeta potential. The emulsion stabilizer is the octanoic acid. The organosiloxane precursors are preferentially a combination of TMAPS, C8-TES, $PEG_{6-9}$-TMS and TEOS in the molar percent range of respectively 10%-22.5%/5%-7.5%/0.5%-10%/85%-70% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm. The negative zeta potential increases from -50 mV (unmodified submicron/nanoparticles) to -30 mV.

In one embodiment, the spheroidal organosiloxane submicron/nanoparticles are synthesized following the general procedure without active/payload, with the help of an emulsion stabilizer, with PEGylation of the external surface and with introduction a -SH function in the organosiloxane matrix. The emulsion stabilizer is the octanoic acid. The organosiloxane precursors are preferentially a combination of SH-TES, C8-TES, $PEG_{6-9}$-TMS and TEOS in the molar percent range of respectively 10%-50%/0%-7.5%/0%-10%/85%-0% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane submicron/nanoparticles present an average particles size between 50 nm and 800 nm.

In one embodiment, the spheroidal organosiloxane submicron/nanoparticles are synthesized following the general procedure without active/payload, with the help of an emulsion stabilizer, with PEGylation of the external surface and with introduction a thiol function in the organosiloxane matrix. The emulsion stabilizer is the octanoic acid. The organosiloxane precursors are preferentially a combination of mercaptopropyltriethoxysilane (SH-TES), C8-TES, PEG6-9-TMS and TEOS in the molar percent range of respectively 10%-50%/0%-7.5%/0%-10%/85%-0% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload, with the help of an emulsion stabilizer, with PEGylation of the external surface and with introduction a thiol function in the organosiloxane matrix. The emulsion stabilizer is the octanoic acid. The organosiloxane precursors are preferentially a combination of SH-TES, C8-TES, $PEG_{6-9}$-TMS and TEOS in the molar percent range of respectively 10%-100%/0%-7.5%/0%-10%/85%-0% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm.

In one embodiment, the spheroidal organosiloxane submicron/nanoparticles are synthesized following the general procedure without active/payload, with the help of an emulsion stabilizer, with PEGylation of the external surface and with introduction a thiol function in the organosiloxane matrix. The emulsion stabilizer is the octanoic acid. The organosiloxane precursors are preferentially a combination of SH-TES, C8-TES, $PEG_{6-9}$-TMS and TEOS in the molar percent range of respectively 10%-100%/0%-7.5%/0%-10%/85%-0% and preferentially the molar percent composition of 21.6%/7.2%/4.0%/67.2%. The pre-condensed organosiloxane precursor's phase is resulted following partial or total removal of volatile solvents. The octanoic acid oil is added to the pre-condensed organosiloxane precursor's phase at a weight ratio from 25 wt% to 50 wt% and preferentially 33 wt%. The condensation catalyst is TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size between 50 nm and 800 nm.

In one embodiment, the spheroidal organosiloxane submicron/nanoparticles are synthesized following the general procedure with active/payload and with the help of an emulsion stabilizer. The emulsion stabilizer is the octanoic acid. The active/payload is a hydrophobic active. The active/payload is $\alpha$-pinene. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the pre-condensed organosiloxane precursor's phase at a weight ratio from 25 wt% to 50 wt% and preferentially 33 wt%. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane submicron/nanoparticles present an average particles size from 50 nm to 800 nm with $\alpha$-pinene loading capacity from 35 wt% to 65 wt% and preferentially 50 wt%.

In one embodiment, the spheroidal organosiloxane submicron/nanoparticles are synthesized following the general procedure with active/payload and with the help of an emulsion stabilizer. The emulsion stabilizer is the octanoic acid. The active/payload is a hydrophobic active. The active/payload is $\alpha$-pinene. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the pre-condensed organosiloxane precursor's phase at a weight ratio 25 wt% to 50 wt% and preferentially 33 wt%. The octanoic acid oil is added to the pre-condensed organosiloxane precursor's phase at a weight ratio from 25 wt% to 50 wt% and preferentially 33 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size from 50 nm to 800 nm with $\alpha$-pinene loading capacity 35 wt% to 65 wt% and preferentially 50 wt%.

In one embodiment, the spheroidal organosiloxane submicron/nanoparticles are synthesized following the general procedure with active/payload and with the help of an emulsion stabilizer. The emulsion stabilizer is the octanoic acid. The active/payload is a hydrophobic active. The active/payload is $\alpha$-pinene. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the pre-condensed organosiloxane precursor's phase at a weight ratio from 25 wt% to 50 wt% and preferentially 33 wt%. The octanoic acid oil is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with $\alpha$-pinene loading capacity from 35 wt% to 65 wt% and preferentially 50 wt%.

In one embodiment, the spheroidal organosiloxane submicron/nanoparticles are synthesized following the general procedure with active/payload and with the help of an emulsion stabilizer. The emulsion stabilizer is the octanoic acid. The active/payload is a hydrophobic active. The active/payload is $\alpha$-pinene. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the pre-condensed organosiloxane precursor's phase at a weight ratio from 25 wt% to 50 wt% and preferentially 33 wt%. The octanoic acid oil is added to the pre-condensed organosiloxane precursor's phase at a weight ratio from 25 wt% to 50 wt% and preferentially 33 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with $\alpha$-pinene loading capacity from 35 wt% to 65 wt% and preferentially 50 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane sub-micron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high force sonicator equipment at a power from 20 W to 400 W and preferentially 50 W. The resulted spheroidal organosiloxane submicron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the oleic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the oleic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane submicron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the oleic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the oleic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high force sonicator equipment at a power from 20 W to 400 W and preferentially 50 W. The resulted spheroidal organosiloxane submicron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the oleic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the oleic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is benzophenone. The emulsion stabilizer is the oleic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the oleic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a benzophenone loading capacity from 15 wt% to 50 wt% and preferentially 32 wt%.

In one embodiment, the spheroidal organosiloxane submicron/nanoparticles are synthesized following the general procedure with active/payload and with the help of an emulsion stabilizer. The active/payload is a hydrophobic active. The active/payload is vitamin A. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane sub-micron/nanoparticles present an average particles size from 50 nm to 800 nm.

In one embodiment, the spheroidal organosiloxane submicron/nanoparticles are synthesized following the general procedure with active/payload and with the help of an emulsion stabilizer. The active/payload is a hydrophobic active. The active/payload is vitamin A. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 33 wt%. The condensation catalyst is an organic base, preferentially TEA. The emulsion is obtained by high force sonicator equipment at a power from 20 W to 400 W and preferentially 50 W. The resulted spheroidal organosiloxane submicron/nanoparticles present an average particles size from 50 nm to 800 nm.

In one embodiment, the spheroidal organosiloxane submicron/nanoparticles are synthesized following the general procedure with active/payload and with the help of an emulsion stabilizer. The active/payload is a hydrophobic active. The active/payload is vitamin A. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 33 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm.

In one embodiment, the spheroidal organosiloxane submicron/nanoparticles are synthesized following the general procedure with active/payload and with the help of an emulsion stabilizer. The active/payload is a hydrophobic active. The active/payload is vitamin A. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 33 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm.

In one embodiment, the spheroidal organosiloxane submicron/nanoparticles are synthesized following the general procedure with active/payload, with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, $PEG_{6-9}$-TMS and TEOS in the molar percent range of respectively 10%-22.5%/5%-7.5%/0,5%-10%/85%-70% and preferentially the molar percent composition of 22.3%/7.4%/1%/69.3%. The pre-condensed organosiloxane precursor's phase is resulted following partial or total removal of volatile solvents. The active/payload is solubilised in the octanoic acid oil at a weight percent from 25 to 50 and preferably 25. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane sub-micron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, $PEG_{6-9}$-TMS and TEOS in the molar percent range of respectively 10%-22.5%/5%-7.5%/0,5%-10%/85%-70% and preferentially the molar percent composition of 22.3%/7.4%/1%/69.3%.The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, $PEG_{6-9}$-TMS and TEOS in the molar percent range of respectively 10%-22.5%/5%-7.5%/0,5%-10%/85%-70% and preferentially the molar percent composition of 22.3%/7.4%/1%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane submicron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, $PEG_{6-9}$-TMS and TEOS in the molar percent range of respectively 10%-22.5%/5%-7.5%/0,5%-10%/85%-70% and preferentially the molar percent composition of 22.3%/7.4%/1%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane submicron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload, with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, octadecyltriethoxysilane (C18-TES), $PEG_{6-9}$-TMS and TEOS in the molar percent range of respectively 10%-22.5%/5%-7.5%/0,5%-10%/85%-70% and preferentially the molar percent composition of 22.6%/7.3%/0.8%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane submicron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C18-TES, $PEG_{6-9}$-TMS and TEOS in the molar percent range of respectively 10%-22.5%/5%-7.5%/0,5%-10%/85%-70% and preferentially the molar percent composition of 22.6%/7.3%/0.8%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size from 50 nm to 800 nm with a benzophenone loading from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C18-TES, $PEG_{6-9}$-TMS and TEOS in the molar percent range of respectively 10%-22.5%/5%-7.5%/0,5%-10%/85%-70% and preferentially the molar percent composition of 22.6%/7.3%/0.8%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane submicron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C18-TES, PEG$_{6-9}$-TMS and TEOS in the molar percent range of respectively 10%-22.5%/5%-7.5%/0,5%-10%/85%-70% and preferentially the molar percent composition of 22.6%/7.3%/0.8%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane submicron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload, with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, mPEG$_{5K}$-silane (PEG$_{5K}$-TES) and TEOS in the molar percent range of respectively 10%-22.5%/5%-7.5%/0.01%-0.1%/85%-70% and preferentially the molar percent composition of 22.3%/7.4%/0.04%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated NH$_4$OH. The emulsion is obtained by high force sonicator equipment at a power from 20 W to 400 W and preferentially 50 W. The resulted spheroidal organosiloxane submicron/nanoparticles present an average particles size from 50 nm to 800 nm with a benzophenone loading from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, PEG$_{5K}$-TES and TEOS in the molar percent range of respectively 10%-22.5%/5%-7.5%/0.01%-0.1%/85%-70% and preferentially the molar percent composition of 22.3%/7.4%/0.04%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, PEG$_{5K}$-TES and TEOS in the molar percent range of respectively 10%-22.5%/5%-7.5%/0.01%-0.1%/85%-70% and preferentially the molar percent composition of 22.3%/7.4%/0.04%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated NH$_4$OH. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload and with the help of an emulsion stabilizer and with PEGylation of the external surface. The active/payload is a hydrophobic active. The active/payload is benzophenone. The emulsion stabilizer is the octanoic acid. The organosiloxane precursors are preferentially a combination of C1-TES, C8-TES, PEG$_{5K}$-TES and TEOS in the molar percent range of respectively 10%-22.5%/5%-7.5%/0.01%-0.1%/85%-70% and preferentially the molar percent composition of 22.3%/7.4%/0.04%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 25 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane submicron/-nanoparticles present an average particles size from 50 nm to 800 nm with a benzophenone loading capacity from 15 wt% to 50 wt% and preferentially 32 wt%. The zeta potential in PBS is in the range of 0-30 mV.

In one embodiment, the spheroidal organosiloxane submicron/nanoparticles are synthesized following the general procedure with active/payload, with the help of an emulsion stabilizer, with PEGylation of the external surface and with introduction of a fluorous function in the organosiloxane matrix. The active/payload is a hydrophobic active. The active/payload is benzophenone. The emulsion stabilizer is the octanoic acid. The organosiloxane precursors are preferentially a combination of trimethoxy(3,3,3-trifluoropropyl)silane ($CF_3$-TMS), C8-TES, $PEG_{6-9}$-TMS and TEOS in the molar percent range of respectively 10%-22.5%/5%-7.5%/0,5%-10%/85%-70% and preferentially the molar percent composition of 21.6%/7.2%/4%/67.2%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 25 /wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane submicron/nanoparticles present an average particles size from 50 nm to 800 nm with a benzophenone loading capacity from 15 % to 50 %.

In one embodiment, the spheroidal organosiloxane submicron/nanoparticles are synthesized following the general procedure with active/payload and with the help of an emulsion stabilizer, with PEGylation of the external surface and with introduction of a fluorous function in the organosiloxane matrix. The active/payload is a hydrophobic active. The active/payload is benzophenone. The emulsion stabilizer is the octanoic acid. The organosiloxane precursors are preferentially a combination of $CF_3$-TMS, C8-TES, $PEG_{6-9}$-TMS and TEOS in the molar percent range of respectively 10%-22.5%/5%-7.5%/0,5%-10%/85%-70% and preferentially the molar percent composition of 22.6%/7.3%/0.8%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high force sonicator equipment at a power from 20 W to 400 W and preferentially 50 W. The resulted spheroidal organosiloxane sub-micron/nanoparticles present an average particles size from 50 nm to 800 nm with a benzophenone loading capacity from 15 % to 50 %.

In one embodiment, the spheroidal organosiloxane submicron/nanoparticles are synthesized following the general procedure with active/payload and with the help of an emulsion stabilizer, with PEGylation of the external surface and with introduction of a fluorous function in the organosiloxane matrix. The active/payload is a hydrophobic active. The active/payload is benzophenone. The emulsion stabilizer is the octanoic acid. The organosiloxane precursors are preferentially a combination of $CF_3$-TMS, C8-TES, $PEG_{6-9}$-TMS and TEOS in the molar percent range of respectively 10%-22.5%/5%-7.5%/0,5%-10%/85%-70% and preferentially the molar percent composition of 22.6%/7.3%/0.8%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 25 wt%. The condensation catalyst is an inorganic base, preferentially concentrated $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a benzophenone loading capacity from 15 % to 50 %.

In one embodiment, the spheroidal organosiloxane submicron/nanoparticles are synthesized following the general procedure with active/payload and with the help of an emulsion stabilizer, with PEGylation of the external surface and with introduction of a fluorous function in the organosiloxane matrix. The active/payload is a hydrophobic active. The active/payload is benzophenone. The emulsion stabilizer is the octanoic acid. The organosiloxane precursors are preferentially a combination of $CF_3$-TMS, C8-TES, $PEG_{6-9}$-TMS and TEOS in the molar percent range of respectively 10%-22.5%/5%-7.5%/0,5%-10%/85%-70% and preferentially the molar percent composition of 22.6%/7.3%/0.8%/69.3%. The pre-condensed organosiloxane precursor's phase is resulted following partial or total removal of volatile solvents. The active/payload is solubilised in the octanoic acid oil at a weight percent from 25 wt% to 50 wt% and preferably 33 wt%. Then the mixture is added to the pre-condensed organosiloxane precursor's phase at a weight ratio from 25 wt% to 50 wt% and preferentially 25 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane submicron/nanoparticles present an average particles size from 50 nm to 800 nm with a benzophenone loading capacity from 15 % to 50 %.

In one embodiment, the spheroidal organosiloxane submicron/nanoparticles are synthesized following the general procedure with active/payload, with the help of an emulsion stabilizer and with an active co-solvent. The active/payload is a hydrophobic active. The active/payload is Paclitaxel. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The paclitaxel is solubilised in the co-solvent, preferentially the diethylene glycol monoethylether (DGME) or triacetin or 2-pentanol, at a weight percent from 1 wt% to 10 wt% and preferentially 10 wt%. Then the solution is solubilised or dispersed in the pre-condensed phase at a weight percent from 25 wt% to 50 wt% and preferably 33 wt%. Then the octanoic acid is added in the dispersed phase at a weight ratio from 25 wt% to 50 wt% and preferentially 25 wt%. The condensation catalyst is an inorganic base, preferentially concentrated $NH_4OH$. The emulsion is obtained by high force sonicator equipment at a power from 20 W to 400 W and preferentially 50 W. The resulted spheroidal organosiloxane submicron/nanoparticles present an average particles size from 50 nm to 800 nm with a paclitaxel loading capacity from 1 % to 10 % and preferentially 6 %.

In one embodiment, the spheroidal organosiloxane submicron/nanoparticles are synthesized following the general procedure with active/payload, with the help of an emulsion stabilizer and with an active co-solvent. The active/payload is a hydrophobic active. The active/payload is Paclitaxel. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The paclitaxel is solubilised in the co-solvent, preferentially DGME or triacetin or 2-pentanol, at a weight percent from 1 wt% to 10 wt% and preferentially 10 wt%. Then the solution is solubilised or dispersed in the pre-condensed phase at a weight percent from 25 wt% to 50 wt% and preferably 33 wt%. Then the octanoic acid is added in the dispersed phase at a weight ratio from 25 wt% to 50 wt% and preferentially 25 wt%. The condensation catalyst is an inorganic base, preferentially TEA. The emulsion is obtained by high force sonicator equipment at a power from 20 W to 400 W and preferentially 50 W. The resulted spheroidal organosiloxane submicron/nanoparticles present an average particles size from 50 nm to 800 nm with a paclitaxel loading capacity from 1 % to 10 % and preferentially 6 %.

In one embodiment, the spheroidal organosiloxane submicron/nanoparticles are synthesized following the general procedure with active/payload, with the help of an emulsion stabilizer and with an active co-solvent. The active/payload is a hydrophobic active. The active/payload is Paclitaxel. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The paclitaxel is solubilised in the co-solvent, preferentially DGME or triacetin or 2-pentanol, at a weight percent from 1 wt% to 10 wt% and preferentially 10 wt%. Then the solution is solubilised or dispersed in the pre-condensed phase at a weight percent from 25 wt% to 50 wt% and preferably 33 wt%. Then the octanoic acid is added in the dispersed phase at a weight ratio from 25 wt% to 33 wt% and preferentially 25 wt%. The condensation catalyst is an organic base, preferentially $NH_4OH$. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a paclitaxel loading capacity from 1 % to 10 %.

In one embodiment, the spheroidal organosiloxane submicron/nanoparticles are synthesized following the general procedure with active/payload, with the help of an emulsion stabilizer and with an active co-solvent. The active/payload is a hydrophobic active. The active/payload is Paclitaxel. The emulsion stabilizer is the octanoic acid. The organosiloxane precursors are preferentially a combination of C1-TES, C8-TES and TEOS in the molar percent range of respectively 10%-22.5%/5%-7.5%/85%-70% and preferentially the molar percent composition of 22.5%/7.5%/70%. The pre-condensed organosiloxane precursor's phase is resulted following partial or total removal of volatile solvents. The paclitaxel is solubilised in the co-solvent, preferentially DGME or triacetin or 2-pentanol, at a weight percent from 1 wt% to 10 wt% and preferentially 10 wt%. Then the solution is solubilised or dispersed in the pre-condensed phase at a weight percent from 25 wt% to 50 wt% and preferably 33 wt%. Then the octanoic acid is added in the dispersed phase at a weight ratio from 25 wt% to 50 wt% and preferentially 25 wt%. The condensation catalyst is an organic base, preferentially TEA. The emulsion is obtained by high shear mixer at preferentially speed rate from 10 000 RPM to 25 000 RPM and preferentially 18 000 RPM. The resulted spheroidal organosiloxane sub-micron/nanoparticles present an average particles size from 50 nm to 800 nm with a paclitaxel loading capacity from 1 % to 10 %.

The present invention also relates to the embodiments listed below.

Embodiment 1: A process of preparation of spheroidal organosiloxane sub-micron/nanoparticles comprising:

i1) separately hydrolyzing at least one organosiloxane precursor in a hydrolytic media to provide one or more pre-hydrolyzed organosiloxane precursor;

i2) combining the pre-hydrolyzed organosiloxane precursors of step i1) to provide a combined pre-hydrolyzed organosiloxane precursor;

i3) removing a part or totality of volatile solvents from said combined pre-hydrolyzed organosiloxane precursors to provide a dispersed phase comprising pre-condensed organosiloxane precursors;

i4) emulsifying, in absence of a surfactant and with shear force or sonication, the dispersed phase of the step i3) in an aqueous continuous phase to provide an oil in water nanoemulsion; and

i5) adding a condensation catalyst to the nanoemulsion of step i4) to obtain the spheroidal organosiloxane submicron/nanoparticles suspension.

Embodiment 2. The process of embodiment 1, wherein the organosiloxane precursors has the formula $R_{4-x}Si(L)_x$ or formula $(L)_3Si-R'-Si(L)_3$, wherein:

R: is a mono-valent residue of an alkyl, alkenyl, alkynyl, alicyclic, aryl, alkyl-aryl group, which is optionally substituted by a halogen atom, glycidyloxy-, -OH, -SH, polyethylene glycol (PEG), $-N(R_a)_2$, or $-N^+(R_a)_3$;

L: is a halogen or an acetoxide $-O-C(O)R_a$, or alkoxide $OR_a$ group;

R': is a bi-valent residue of an alkyl, alkenyl, alkynyl, alicyclic, aryl, alkyl-aryl group, which is optionally substituted by a halogen atom, -OH, -SH, $-N(R_a)_2$, or $-N^+(R_a)_3$;

$R_a$ can each independently be hydrogen, alkyl, alkenyl, alkynyl, alicyclic, aryl or alkyl-aryl; and

X: is an integer of 1 to 4 or alternatively x is an integer of 1 to 3.

Embodiment 3. The process of embodiment 1, wherein in step i2) a nanoemulsion stabilizer is added in the combined pre-hydrolyzed organosiloxane precursor.

Embodiment 4. The process of embodiment 1, wherein in Step i3), a non-hydrolyzed organosiloxane precursor, other than said organosiloaxe precursors in i1), is added in the dispersed phase.

Embodiment 5. The process of embodiment 1, step i3), wherein a hydrolyzed organosiloxane precursor, other than said organosiloxane precursors in i1), is added in the dispersed phase.

Embodiment 6. The process of embodiment 1, wherein in step i3) an active/payload insoluble in the continuous phase is added in the dispersed phase.

Embodiment 7. The process of embodiment 3, wherein said nanoemulsion stabilizer is a carboxylic acid- containing compound comprising at least 8 carbon atoms.

Embodiment 8. The process of embodiment 6, wherein said active/payload insoluble in the continuous phase, is a hydrophobic/liposoluble molecule in a liquid state.

Embodiment 9. The process of embodiment 6, wherein said active/payload insoluble in the continuous phase, is a hydrophobic molecule in a solid state.

Embodiment 10. The process of any one of embodiments 6 and 8, wherein said active/payload insoluble in the continuous phase, is a cosmetic, cosmeceutical or pharmaceutical compound.

Embodiment 11. The process of embodiments 6 and 8 to 10, wherein said active/payload is a Taxane.

Embodiment 12. The process of embodiment 8, wherein said nanoemulsion stabilizer is octanoic acid.

Embodiment 13. A spheroidal organosiloxane submicron/nanoparticle comprising a network consisting of organosiloxane, wherein said spheroidal organosiloxane submicron/nanoparticle is uncalcined amorphous, surfactant-free and is nano to submicron size, said particle optionally comprising an active/payload.

Embodiment 14. The spheroidal organosiloxane submicron/nanoparticle as defined in embodiment 13,

wherein said spheroidal organosiloxane sub-micron/nanoparticles are nano to submicron size;

wherein said spheroidal organosiloxane sub-micron/nanoparticles are porous when an active/payload is sequestered and are nonporous when prepared without active/payload;

wherein said porosity of said spheroidal organosiloxane submicron/nanoparticles is assessed by pore volume, pore diameter and specific surface area as measured by $N_2$ physisorption;

wherein said active/payload is insoluble in the aqueous continuous phase.

Embodiment 15. A spheroidal organosiloxane submicron/nanoparticle prepared by the process as defined in any one of embodiments 1 to 12.

Embodiment 16. A method for in-situ sequestration of an active/payload throughout the spheroidal organosiloxane submicron/nanoparticles as defined in embodiment 13, 14 or 15, comprising incorporating said active/payload in said submicron/nanoparticles by a process as defined in any one of embodiments 6 and 8 to 11.

Embodiment 17. A method for modulating the release of an active/payload, comprising incorporating said active/payload in spheroidal organosiloxane submicron/nanoparticles as defined in any one of embodiments 13 to 15, or incorporating said active/payload in a process as defined in any one of embodiments 1 to 12.

## SAMPLE CHARACTERIZATION

**Scanning Electron Microscopy (SEM):** SEM images of the spheroidal organosiloxane sub-micron/nanoparticles were recorded with FEI Quanta-3D-FEG at 3.0 kV without coating or with JEOL 840-A at 15 kV with gold coating.
**Particles size distribution:** To measure the particle size distribution, Dynamic Light Scattering (DLS) analysis and Malvern Mastersizer analysis were used:

• **Dynamic light scattering (DLS):** The hydrodynamic diameter of the spheroidal organosiloxane sub-micron/nanoparticles and their colloidal stability was monitored by dynamic light scattering (DLS) with a Malvern DTS Nano zetasizer 1731 (T = 25 °C, equilibration time set to 3 min; 2 or 3 measurements was taken on each sample; only quality criteria data accepted as valid results). Prior to analyses, 10 mg of spheroidal organosiloxane submicron/nanoparticles powder were dispersed in 10 mL of water with an ultrasonic bath (10 min) and a vortex (1 min). The resulting suspension was diluted to meet the concentration criteria of DLS analyses. Solutions prepared for DLS need to be clear to very slightly hazy to avoid a lack of accurate due to multiple scattering or viscosity effects.

• **Malvern Mastersizer analysis:** The negative control silica particles (the obtained microspheres, about 50 mg) was dispersed in methanol of about 5 mL in ultrasonic bath for 5 minutes to obtain a well dispersed solution, which was then added into the sonicated bath of Malvern Mastersizer 2000 (Hydro 2000S, Model AWA2001) till the obstruction of the signal was about 5 to 8%.

**Specific surface area (BET) and porosity:** The surface area and porosity of the spheroidal organosiloxane submicron/nanoparticles were characterized with Micrometrics TriStar™ 3000 V4.01 and Micrometrics TriStar™ 3020 V3.02 at 77 K. The collected data were analyzed using the standard Brunauer-Emmett-Teller (BET) to get the surface area, and the pore size was obtained from the maxima of the pore size distribution curve calculated by Barrett-Joyner-Halenda (BJH) method using the adsorption branch of the isotherm.
**Active Quantification in** spheroidal organosiloxane submicron/nanoparticles -: The loading of actives sequestered in spheroidal organosiloxane sub-micron/nanoparticles were determined by suspending certain amount (generally 100 mg) of sequestered spheroidal organosiloxane sub-micron/nanoparticles in 10 mL of a 10% ammonia aqueous solution, which was then sonicated in Branson 8800 ultrasonic bath for 30 minutes, and followed by 2 hours shaking with using IKA HS-501 Horizontal shaker at 200 mot/min to achieve fully release. The spheroidal organosiloxane submicron/nanoparticles were filtered off through a 0.22 $\mu$m filter to give a clear solution for HPLC analysis.
**Active concentration measurement:** - Active concentration was determined in this solution, using HPLC technique (Agilent 1100 equipped with a quaternary solvent delivery system (G1311A), vacuum degasser unit (G1322A), UV photodiode array detector (G1314A), standard autosampler (G1313A) and thermostatic column compartment (G1316A)). The column used herein was the SiliaChrom DtC18 column of 3 X 150 mm i.d., 5 $\mu$m, 100 Å. 0.1 % formic acid containing water was used as the mobile phase MPA and 0.1 % formic acid containing acetonitrile was used as the mobile phase MPB. The injections volume was 2 μL. The Starting mobile phase was 95 % MPA and 5 % MPB, and ends at 95 % MPB at 4 minutes, hold for another 2 minutes. The flow rate, column temperature and the detector were set at 0.5 ml.min$^{-1}$, 23 °C and 260 nm respectively. Uracil retention time is 1.88 min, benzophenone retention time is 1.78 min and paclitaxel retention time is 3.20 min. The calibration curves were constructed with pure compounds purchased from Sigma Aldrich.

**Water Quantification in spheroidal organosiloxane submicron/nanoparticles (Karl Fisher):** The water percentage was estimated by using titrator Compact V20s from Mettler Toledo.

**Zeta potential:** To determine the Zeta potential of the spheroidal organosiloxane sub-micron/nanoparticles, 10 mg of spheroidal organosiloxane sub-micron/nanoparticles powder were dispersed in 10 mL of water with an ultrasonic bath (10 min) and a vortex (1 min). The resulting suspension was diluted to meet the concentration criteria of the analyses. The suspension was placed in a Capillary Zeta Cell for the zeta potential measurement with Malvern, Zetasizer Nano ZS.

**X-ray Photoelectron Spectroscopy (XPS):** The chemical composition of the external surface was investigated in a maximum depth of 5 nanometers by X-ray photoelectron spectroscopy, using Axis-Ultra de Kratos (UK). The main XPS chamber was maintained at a base pressure of < $5.10^{-8}$ Torr. A monochromatic aluminum X-ray source (Al k$\alpha$ = 1486.6 eV) at 250 W was used to record survey spectra (1400-0 eV) and high-resolution spectra with charge neutralization. The detection angle was set at 45° with respect to the normal of the surface and the analyzed area was 0.016 cm$^2$ (aperture 5).

**EXAMPLES**

**Spheroidal organosiloxane sub-micron/nanoparticles were produced without using stabilizer**

**Example 1-1:** Organosiloxane sub-micron/nanoparticles were produced by using rotavapor for pre-condensed phase preparation and turrax mixer for the emulsification. Initial molar composition of the precursors: 22.5% C$_1$-TES, 7.5% C$_8$-TES and 70% TEOS.

A 150 mL round bottle flask was first charged with 0.16 g of 0.01 M hydrochloric acid and 0.75 g of ethanol, followed by the addition of 1.72 g (8.3 mmol) of tetraethyl orthosilicate (TEOS). The molar ratio of H$_2$O to TEOS was 1.1:1. In one 30 mL vial, 0.48 g (2.7 mmol) of methyl triethoxysilane (C$_1$-TES) was mixed with 0.16 g of 0.01 M hydrochloric acid. The molar ratio of H$_2$O to C$_1$-TES was 3.3:1. In another 30 mL vial, 0.24 g (0.9 mmol) of n-octyltriethoxylsilane (C$_8$-TES) was combined with respectively 0.53 g of 0.05 M hydrochloric acid, as well as 0.5 g of THF. The molar ratio of H$_2$O to C$_8$-TES was 3.3:1. The above three mixtures were stirred for 1.5 hour and were subsequently combined in the 150 mL round bottle flask. The volatile solvents in the hydrolyzed organosiloxane precursors were gradually distilled at 40 °C under reduced pressure to pre-condense the organosiloxane precursors. To produce the O/W emulsion, 150 mL of distilled water, in a separate container, was stirred with Ultra-Turrax homogenizer at 18K rpm, and the 0.75g of the dispersed phase was then added. After continuous stirring for 5 min at 18K rpm, 6.0 ml of triethanolamine (TEA) was introduced in the emulsion dropwise as condensation catalyst while stirring. The mixing was continued for 1 min. The resulting suspension was further aged at room temperature in an oscillating stirrer at the speed of 200 rpm overnight. The product was centrifuged at 20 G for 10 min, thoroughly washed with distilled water to remove residual TEA and dried at room temperature to obtain the organosiloxane submicron/nanoparticles. The diameter of organosiloxane submicron/nanoparticles, measured by dynamic light scattering (DLS) analysis in intensity mode, showed the average hydrodynamic particle size (Z-average) of 223 nm and polydispersity index (PDI) of 0.148.

**Example 1-2** Organosiloxane sub-micron/nanoparticles were produced by using distillation for pre-condensed phase preparation and ultrasonication for the emulsification. Initial molar composition of the precursors: 22.5% C$_1$-TES, 7.5% C$_8$-TES and 70% TEOS.

A 150 mL round bottle flask was first charged with 0.16 g of 0.01 M hydrochloric acid and 0.75 g of ethanol, followed by the addition of 1.72 g (8.3 mmol) of tetraethyl orthosilicate (TEOS). The molar ratio of H$_2$O to TEOS was 1.1:1. In one 30 mL vial, 0.48 g (2.7 mmol) of methyl triethoxysilane (C$_1$-TES) was mixed with 0.16 g of 0.01 M hydrochloric acid. The molar ratio of H$_2$O to C$_1$-TES was 3.3:1. In another 30 mL vial, 0.24 g (0.9 mmol) of n-octyltriethoxylsilane (C$_8$-TES) was combined with respectively 0.53 g of 0.05 M hydrochloric acid, as well as 0.5 g of THF. The molar ratio of H$_2$O to C$_8$-TES was 3.3:1. The above three mixtures were stirred for 1 hour and were subsequently combined in the 150 mL round bottle flask to form the hydrolyzed organosiloxane precursors mixture. The volatile solvents in the hydrolyzed organosiloxane precursors were distilled at 101 °C at atmospheric pressure to form the pre-condensed phase. 0.82 g of $\alpha$-Pinene was added to the pre-condensed phase to form the dispersed phase. To produce the O/W emulsion, 40 mL of distilled water, in a separate container, was emulsified with a ultrasonification process at 25 W and 0.5 g of the dispersed phase was then added. After discontinuous sonication (50% time on/off) for 4 min at 25 W, 0.5 ml of concentrated ammoniac was introduced in the emulsion dropwise as condensation catalyst while stirring. The mixing was continued for 45 s. The resulting suspension was further aged at room temperature in an oscillating stirrer at the speed of 200 rpm overnight. The product was centrifuged at 20 G for 10 min, thoroughly washed with distilled water to remove residual ammoniac and dried at room temperature to obtain the organosiloxane submicron/nanoparticles. The diameter of organosiloxane submicron/nanoparticles, measured by dynamic light scattering (DLS) analysis in intensity mode, showed a population with a hydrodynamic particle size of 255 nm and important polydispersity index (PDI) of 0.823.

Spheroidal organosiloxane sub-micron/nanoparticles were produced with stabilizer

**EXAMPLE 2:** Spheroidal organosiloxane submicron/nanoparticles produced with octanoic acid, using rotavapor for pre-condensed phase preparation and turrax mixer for the emulsification step. Initial molar composition of the precursors: 22.5% $C_1$-TES, 7.5% $C_8$-TES and 70% TEOS.

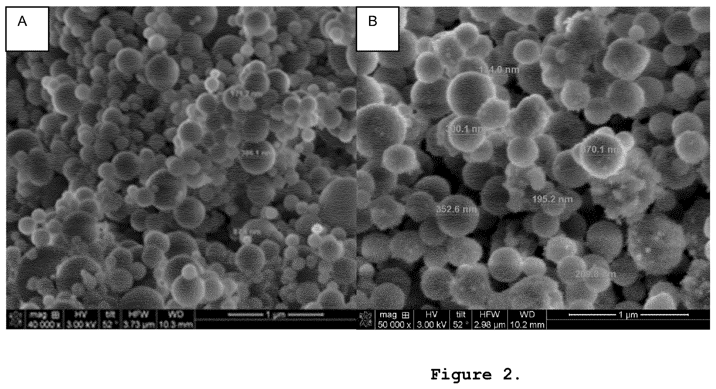

A 150 mL round bottle flask was first charged with 0.16 g of 0.01 M hydrochloric acid and 0.75 g of ethanol, followed by the addition of 1.72 g (8.3 mmol) of tetraethyl orthosilicate (TEOS). The molar ratio of $H_2O$ to TEOS was 1.1:1. In one 30 mL vial, 0.48 g (2.7 mmol) of methyl triethoxysilane ($C_1$-TES) was mixed with 0.16 g of 0.01 M hydrochloric acid. The molar ratio of $H_2O$ to $C_1$-TES was 3.3:1. In another 30 mL vial, 0.24 g (0.9 mmol) of n-octyltriethoxylsilane ($C_8$-TES) was combined with respectively 0.53 g of 0.05 M hydrochloric acid, as well as 0.5 g of THF. The molar ratio of $H_2O$ to $C_8$-TES was 3.3:1. The above three mixtures were stirred for 1.5 hour and were subsequently combined in the 150 mL round bottle flask. The volatile solvents in the hydrolyzed organosiloxane precursors were gradually distilled at 40 °C under reduced pressure to pre-condense the organosiloxane precursors. Followed by the addition of 0.62 g (4.3 mmol) of octanoic acid to achieve the dispersed phase. To produce the O/W emulsion, 250 mL of distilled water, in a separate container, was stirred with Ultra-Turrax homogenizer at 18K rpm, and the entirety of the dispersed phase was then added. After continuous stirring for 5 min at 18K rpm, 2.5 ml of concentrated $NH_4OH$ (28-29%) was introduced in the emulsion dropwise as condensation catalyst while stirring. The mixing was continued for 1 min. The resulting suspension was further aged at room temperature in an oscillating stirrer at the speed of 200 rpm overnight. The product was centrifuged at 20 G for 10 min, thoroughly washed with distilled water to remove residual $NH_4OH$ and dried at room temperature to obtain the spheroidal organosiloxane sub-micron/nanoparticles. The diameter of spheroidal organosiloxane sub-micron/nanoparticles, measured by dynamic light scattering (DLS) analysis in intensity mode, showed the average hydrodynamic particle size (Z-average) of 211 nm and polydispersity index (PDI) of 0.09. The porosity, determined by $N_2$-physisorption isotherms, exhibited the BET surface area of 22 $m^2.g^{-1}$, pore volume of 0.02 $cm^3. g^{-1}$ and pore size of 5.5 nm. The SEM image is presented in Figure 2.

**EXAMPLE 3:** Spheroidal organosiloxane submicron/nanoparticles produced with octanoic acid, using distillation for pre-condensed phase preparation and ultrasonification for the emulsification step. Initial molar composition of the precursors: 22.5% $C_1$-TES, 7.5% $C_8$-TES and 70% TEOS.

Spheroidal organosiloxane sub-micron/nanoparticles were manufactured by following the procedure described in EXAMPLE 2 with the exceptions stated below: 1) The chemicals and the amount used were as in Table 1; 2) The volatile solvents in the hydrolyzed organosiloxane precursors were distilled at 100 °C at atmospheric pressure to form the pre-condensed phase and 3) The emulsification was accomplished with ultrasonic agitation (50 W, 10 min).

The obtained spheroidal organosiloxane submicron/nanoparticles had an average hydrodynamic particle size of 212 nm and PDI of 0.074. The SEM image is presented in Figure 2.

**EXAMPLE 4:** Spheroidal organosiloxane submicron/nanoparticles loaded with benzophenone (BP). Initial molar composition of the precursors: 22.5% $C_1$-TES, 7.5% $C_8$-TES and 70% TEOS.

Sequestration of BP in spheroidal organosiloxane submicron/nanoparticles were manufactured by following the procedure described in EXAMPLE 2 with the exceptions stated below: 1) The chemicals and the amount used are as in table 1; 2) The volatile solvents in the hydrolyzed organosiloxane precursors were distilled at 100 °C at atmospheric pressure for pre-condensation; 3)A proportion of 25 wt % of BP defined as the weight ratio (

$$\frac{m_{BP}}{m_{octanoic\ acid} + m_{BP}} \times 100$$

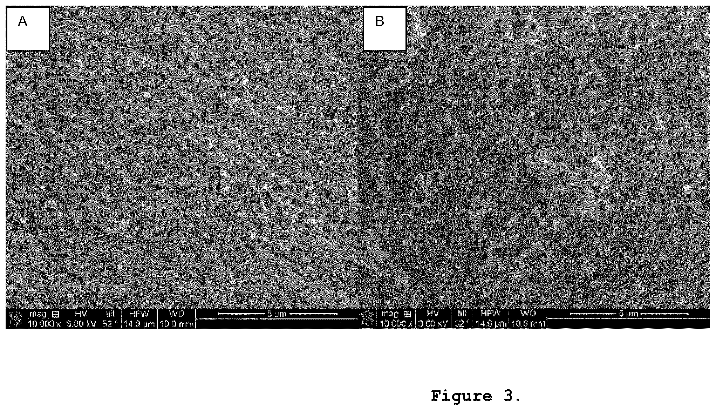

) was solubilised in the octanoic acid oil then added and homogenized with the pre-condensed phase; 4) The emulsification was accomplished with ultrasonic agitation (50 W, 10 min). The final material had an average hydrodynamic particle size of 203 nm and PDI of 0.14. The SEM image is presented in Figure 3.

**EXAMPLE 5:** Spheroidal organosiloxane submicron/nanoparticles loaded BP. Effect of the condensation catalyst: triethanolamine (TEA) instead of concentrated ammonia. Initial molar composition of the precursors: 22.5% $C_1$-TES, 7.5% $C_8$-TES and 70% TEOS.

Sequestration of BP in spheroidal organosiloxane submicron/nanoparticles were manufactured by following the procedure described in EXAMPLE 2 with the exceptions stated below: 1) The chemicals and the amount used in this experiment are as in table 1; 2) The volatile solvents in the hydrolyzed organosiloxane precursors were distilled at 100 °C at atmospheric pressure to form the pre-condensed phase; 3) A proportion of 25 wt % of BP defined as the weight ratio (

$$\frac{m_{BP}}{m_{octanoic\ acid} + m_{BP}} \times 100$$

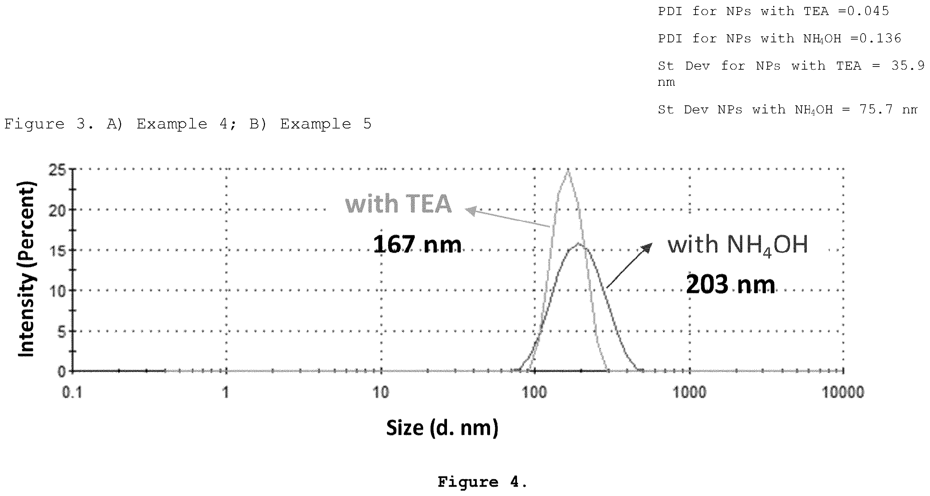

) was solubilised in the octanoic acid oil then added and homogenized with the pre-condensed phase; 4) The emulsification was accomplished with ultrasonic agitation (50 W, 10 min). 5) The triethanolamine (TEA) was used as condensation catalyst. The final material has an average hydrodynamic particle size of 167 nm and PDI of 0.05. The SEM image is presented in Figure 3. The comparison of the spheroidal organosiloane submicron/nanoparticles produced in example 3 and example 4 are presented in Figure 4.

**EXAMPLE 6:** Spheroidal organosiloxane submicron/nanoparticles loaded with BP and further modified with pre-hydrolyzed PEG silane ($PEG_{6-9}$-TMS). Initial molar composition of the precursors: 22.3% C1-TES, 7.4% C8-TES, 1.0 % PEG-TMS (6-9) and 69.3 % TEOS

Sequestration of BP and matrix modification of spheroidal organosiloxane sub-micron/nanoparticles were manufactured by following the procedure described in EXAMPLE 2 with the exceptions stated below: 1) The chemicals and the amount used in this experiment are as in table 1; 2) A proportion of 25 wt % of BP defined as the weight ratio (

$$\frac{m_{BP}}{m_{octanoic\ acid} + m_{BP}} \times 100$$

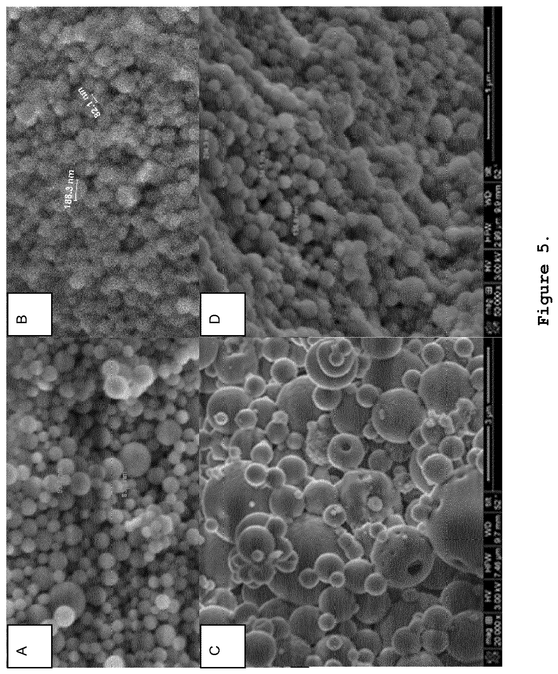

) was solubilised in the octanoic acid oil then added and homogenized with the pre-condensed phase; 3) Triethanolamine was used as condensation catalyst; 4) 2-[methoxy(polyethyleneoxy)6-9 propyl] trimethoxysilane ($PEG_{6-9}$-TMS) was pre-hydrolyzed with HCl (0.01 M) at the $H_2O/PEG_{6-9}$-TMS molar ratio of 4:1 and then mixed with pre-condensed phase. The goal was to modify the external surface of spheroidal organosiloxane nanoparticles. The final material had an average hydrodynamic particle size of 239 nm and PDI of 0.08. The SEM image is presented in Figure 5.

**EXAMPLE 7:** Spheroidal organosiloxane submicron/nanoparticles loaded with BP and further modified with un-hydrolyzed PEG silane ($PEG_{5K}$-TES). Initial molar composition of the precursors: 22.3% $C_1$-TES, 7.2% $C_8$-TES, 0.04 % $PEG_{5K}$-TES and 70.46 % TEOS

Sequestration of BP and matrix modification of spheroidal organosiloxane sub-micron/nanoparticles were manufactured by following the procedure described in EXAMPLE 2 with the exceptions stated below: 1) The chemicals and the amount used in this experiment are as in table 1; 2) A proportion of 25 wt % of BP defined as the weight ratio (

$$\frac{m_{BP}}{m_{octanoic\ acid} + m_{BP}} \times 100$$

) was solubilised in the octanoic acid oil then added and homogenized with the pre-condensed phase; 3) Triethanolamine was used as condensation catalyst; 4) $mPEG_{5K}$-TES was used directly after adding the condensation catalyst in the goal to modify the external surface of spheroidal organosiloxane sub-micron/nanoparticles. The final material had an average hydrodynamic particle size of 200 nm and PDI of 0.15. The SEM image is presented in Figure 5.

**EXAMPLE 8:** Spheroidal organosiloxane submicron/nanoparticles loaded with BP, further modified with PEG silane ($PEG_{6-9}$-TMS) and prepared by using NaOH as condensation catalyst. Initial molar composition of the precursors: 22.0 % $C_1$-TES, 7.5% $C_8$-TES, 2.0 % $PEG_{6-9}$-TMS and 68.5 % TEOS

Sequestration of BP and matrix modification of spheroidal organosiloxane sub-micron/nanoparticles were manufactured with using NaOH as condensation catalyst by following the procedure described in EXAMPLE 2 with the exceptions stated below: 1) The chemicals and the amount used in this experiment are as in table 1; 2) A proportion of

$$\frac{m_{BP}}{m_{octanoic\ acid} + m_{BP}} \times 100$$

25 wt % of BP defined as the weight ratio ( ) was solubilised in the octanoic acid oil then added and homogenized with the pre-condensed phase; 3) NaOH (1M) was used as condensation catalyst; 4) 2-[methoxy(polyethyleneoxy) 6-9 propyl]trimethoxysilane was pre-hydrolyzed with HCl (0.01 M) at the $H_2O/PEG_{6-9}$-TMS molar ratio of 4:1, it was added directly in the pre-condensed phase and used in the goal to modify the external surface of spheroidal organosiloxane submicron/nanoparticles. The final material had an average hydrodynamic particle size of 277 nm and PDI of 0.14. The SEM image is presented in Figure 5.

**EXAMPLE 9:** Spheroidal organosiloxane submicron/nanoparticles loaded with BP, further modified with PEG silane ($PEG_{6-9}$-TMS) and produced by using L-lysine as condensation catalyst. Initial molar composition of the precursors: 22.0 % $C_1$-TES, 7.5% $C_8$-TES, 2.0 % $PEG_{6-9}$-TMS and 68.5 % TEOS.

Sequestration of BP and matrix modification of spheroidal organosiloxane sub-micron/nanoparticles were manufactured with using L-lysine as condensation catalyst by following the procedure described in EXAMPLE 2 with the exceptions stated below: 1) The chemicals and the amount used in this experiment are as in table 1; 2) A proportion of

$$\frac{m_{BP}}{m_{octanoic\ acid} + m_{BP}} \times 100$$

25 wt % of BP defined as the weight ratio ( ) was solubilised in the octanoic acid oil then added and homogenized with the pre-condensed phase; 3) L-lysine was used as condensation catalyst; 4) 2-[methoxy(polyethyleneoxy) 6-9 propyl]trimethoxysilane was pre-hydrolyzed with HCl (0.01 M) at the $H_2O/PEG$-silane molar ratio of 4:1, it was added directly in the pre-condensed phase and used in the goal to modify the external surface of spheroidal organosiloxane submicron/nanoparticles. The final material had an average hydrodynamic particle size of 300 nm and PDI of 0.20. The SEM image is presented in Figure 5.

**EXAMPLE 10:** Spheroidal organosiloxane submicron/nanoparticles loaded with α-pinene. Initial molar composition of the precursors: 22.5% $C_1$-TES, 7.5% $C_8$-TES and 70% TEOS.

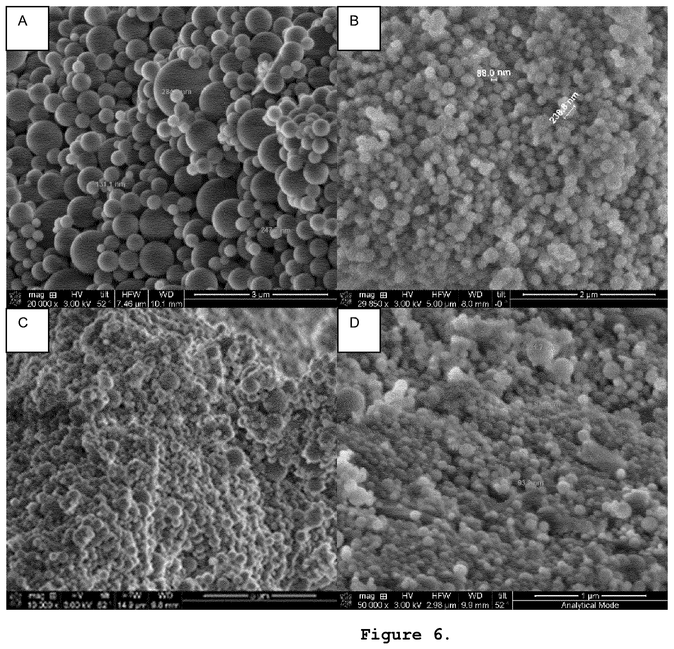

Sequestration of α-pinene in spheroidal organosiloxane sub-micron/nanoparticles were manufactured by following the procedure described in EXAMPLE 2 with the exceptions stated below: 1) The chemicals and the amount used in this experiment are as in table 1; 2) α-pinene was added directly to the dispersed phase and was employed to achieve spheroidal organosiloxane sub-micron/nanoparticles at the loading of 32 wt% of α-pinene. The final material had an average hydrodynamic particle size of 377 nm and PDI of 0.11. The porosity exhibited the BET surface area of 689 $m^2.g^{-1}$, pore volume of 0.80 $cm^3.g^{-1}$ and pore size of 3.9 nm. The SEM image is presented in Figure 6.

**EXAMPLE 11:** Spheroidal organosiloxane submicron/nanoparticles loaded with vitamin A. Initial molar composition of the precursors: 22.5% $C_1$-TES, 7.5% $C_8$-TES and 70% TEOS.

Sequestration of Vitamin A in speroidal organosiloxane sub-micron/nanoparticles were manufactured by following the procedure described in EXAMPLE 2 with the exceptions stated below: 1) The chemicals and the amount used in this experiment are as in table 1; 2) A proportion of 50 wt% of vitamin A defined as the weight ratio (

$$\frac{m_{Vitamine\ A}}{m_{octanoic\ acid} + m_{Vitamine\ A}} \times 100$$

) was solubilised in octanoic acid was employed to achieve spheroidal organosiloxane sub-micron/nanoparticles at the experimental loading of 26 wt% of vitamin A. The final material had an average hydrodynamic particle size of 414 nm and PDI of 0.20. The porosity exhibited the BET surface area of 530 $m^2.g^{-1}$, pore volume of 0.38 $cm^3.g^{-1}$ and pore size of 2.9 nm. The SEM image is presented in Figure 6.

**EXAMPLE 12:** Spheroidal organosiloxane submicron/nanoparticles produced by using triacetin as co-solvent. Initial molar composition of the precursors: 22.5% $C_1$-TES, 7.5% $C_8$-TES and 70% TEOS.

The spheroidal organosiloxane sub-micron/nanoparticles were manufactured using triacetin as a potential co-solvent for the active by following the procedure described in EXAMPLE 2 with the exceptions stated below: 1) The chemicals and the amount used in this experiment are as in table 1; 2) Triacetin was used as potential co-solvent to dissolve actives and was added with the octanoic acid oil in the pre-condensed phase; 3) Triethanolamine was used as a condensation catalyst. The final material had an average hydrodynamic particle size of 190 nm and PDI of 0.19. The SEM image is presented in Figure 6.

**EXAMPLE 13:** Spheroidal organosiloxane submicron/nanoparticles loaded with Paclitaxel, which was dissolved in diethylene glycol monoethylether (DGME) as co-solvent. The octanoic acid was added in the continuous phase prior to the emulsification step. Initial molar composition of the precursors: 22.5% $C_1$-TES, 7.5% $C_8$-TES and 70% TEOS.

Sequestration of Paclitaxel in spheroidal organosiloxane sub-micron/nanoparticles were manufactured by following the procedure described in EXAMPLE 2 with the exceptions stated below: 1) The chemicals and the amount used in this experiment are as in table 1; 2) Octanoic acid was added in the continuous phase prior to the emulsification; 3) 10 wt% of paclitaxel was solubilized in DGME and the 0.5 mL of paclitaxel-DGME solution was mixed to 1 ml of the dispersed phase. 4) 1 mL of octanoic acid was added in the dispersant phase prior to the emulsification 5) Triethanolamine was used as condensation catalyst. The SEM image is presented in Figure 6.

**EXAMPLE 14:** Spheroidal organosiloxane submicron/nanoparticles produced with TEOS and $C_1$-TES as organosiloxane precursors and loaded with BP. Initial molar composition of the precursors: 25 % $C_1$-TES and 75% TEOS.

Spheroidal organosiloxane sub-micron/nanoparticles were manufactured by following the procedure described in EXAMPLE 2 with the exceptions stated below: 1) The chemicals and the amount used in this experiment are as in table 1; 2) The volatile solvents in the hydrolyzed organosiloxane precursors were distilled at 100 °C at atmospheric pressure; 3) A

$$\frac{m_{BP}}{m_{octanoic\ acid} + m_{BP}} \times 100$$

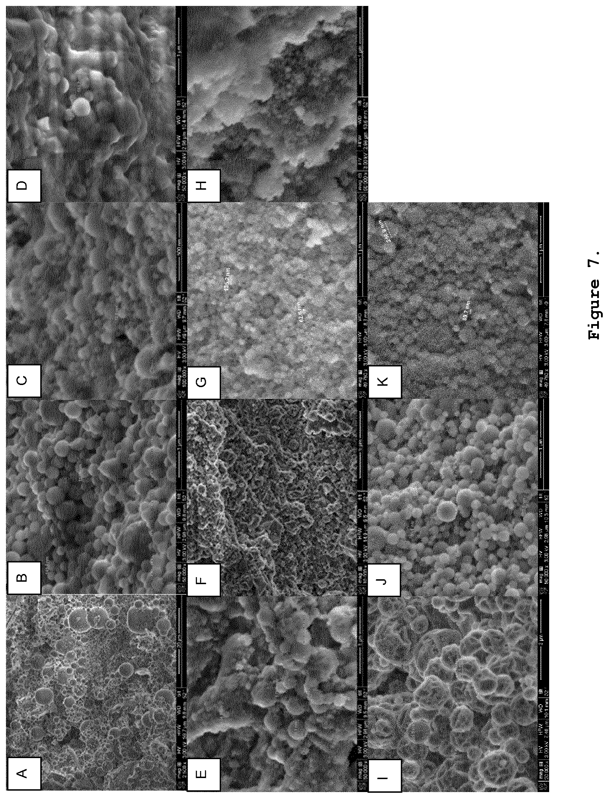

proportion of 25 wt % of BP defined as the weight ratio ( ) was solubilised in the octanoic acid oil then added and homogenized with the pre-condensed phase; 4) Methyl triethoxysilane was used as the solely organosiloxane precursor. The final material had an average hydrodynamic particle size of 366 nm and PDI of 0.21. The porosity exhibited the BET surface area of 521 $m^2.g^{-1}$, pore volume of 0.31 $cm^3.g^{-1}$ and pore size of 2.4 nm. The SEM image is presented in Figure 7.

**EXAMPLE 15:** Spheroidal organosiloxane submicron/nanoparticles produced with $C_{18}$-TES and PEG silane ($PEG_{6-9}$-TMS) and loaded with BP. Initial molar composition of the precursors: 22.6 % $C_1$-TES, 0.8 % $PEG_{6-9}$-TMS, 7.3 % C18-TES and 69.3 % TEOS

Spheroidal organosiloxane sub-micron/nanoparticles were manufactured by following the procedure described in EXAMPLE 2 with the exceptions stated below: 1) The chemicals and the amount used in this experiment are as in table 1; ; 2) A proportion of 25 wt % of BP defined as the weight ratio

$$\frac{m_{BP}}{m_{octanoic\ acid} + m_{BP}} \times 100$$

( ) was solubilised in the octanoic acid oil then added and homogenized with the pre-condensed phase 3) n-Octadecyltriethoxysilane (C18-TES) was used to construct spheroidal organosiloxane sub-micron/nanoparticles, instead of C8-TES; 4) 2-[methoxy(polyethyleneoxy) 6-9 propyl]trimethoxysilane was pre-hydrolyzed with HCl (0.01 M) at the $H_2O$/PEG-silane molar ratio of 4:1, it was added directly in the pre-condensed phase and used in the goal to modify the external surface of spheroidal organosiloxane submicron/nanoparticles. The final material had an average hydrodynamic particle size of 206 nm and PDI of 0.078. The SEM image is presented in Figure 7.

**EXAMPLE 16:** Spreroidal organosiloxane submicron/nanoparticles produced with CF3 groups and PEG silane (PEG$_{6-9}$-TMS). Initial molar composition of the precursors: 21.6 % Trimethoxy(3,3,3-trifluoropropyl)silane (CF$_3$-TMS), 7.2 % C$_8$-TES, 4.0 % PEG$_{6-9}$-TMS and 67.2 % TEOS

Spheroidal organosiloxane sub-micron/nanoparticles were manufactured by following the procedure described in EXAMPLE 2 with the exceptions stated below: 1) The chemicals and the amount used in this experiment are as in table 1; 2) Trimethoxy(3,3,3-trifluoropropyl)silane (CF$_3$-TMS) was pre-hydrolyzed with HCl (0.01 M) at the H$_2$O/CF$_3$-TMS molar ratio of 3:1 and used as one of the organosiloxane precursors; 3) 2-[methoxy(polyethyleneoxy)6-9 propyl]trimethoxysilane was pre-hydrolyzed with HCl (0.01 M) at the H$_2$O/PEG-silane molar ratio of 4:1, it was added directly in the pre-condensed phase and used in the goal to modify the external surface of spheroidal organosiloxane sub-micron/nanoparticles; 4) Triethanolamine was used as condensation catalyst. The SEM image is presented in Figure 7.

**EXAMPLE 17:** Spheroidal organosiloxane submicron/nanoparticles produced with dimethylsilyl (-(CH$_3$)$_2$) groups and PEG silane (PEG$_{6-9}$-TMS). Initial molar composition of the precursors: 21.6 % Dimethyldimethoxysilane (DMDMS), 7.2 % C$_8$-TES, 4.0 % PEG$_{6-9}$-TMS and 67.2 % TEOS.

Spheroidal organosiloxane sub-micron/nanoparticles were manufactured by following the procedure described in EXAMPLE 2 with the exceptions stated below: 1) The chemicals and the amount used in this experiment are as in table 1; 2) Dimethyldimethoxysilane (DMDMS) was pre-hydrolyzed with HCl (0.01 M) at the H$_2$O/DMDMS molar ratio of 3:1, and used as one of the organosiloxane precursors; 3) 2-[methoxy(polyethyleneoxy)6-9 propyl]trimethoxysilane was pre-hydrolyzed with HCl (0.01 M) at the H$_2$O/PEG-silane molar ratio of 4:1, it was added directly in the pre-condensed phase and used in the goal to modify the external surface of spheroidal organosiloxane submicron/nanoparticles; 4) Triethanolamine was used as condensation catalyst. The SEM image is presented in Figure 7.

**EXAMPLE 18:** Spheroidal organosiloxane submicron/nanoparticles produced with Mercaptopropyl groups (-SH) and PEG silane (PEG$_{6-9}$-TMS). Initial molar composition of the precursors: 21.6 % (3-Mercaptopropyl)trimethoxysilane (SH-TMS), 7.2 % C$_8$-TES, 4.0 % PEG$_{6-9}$-TMS and 67.2% TEOS.

Spheroidal organosiloxane sub-micron/nanoparticles were manufactured by following the procedure described in EXAMPLE 2 with the exceptions stated below: 1) The chemicals and the amount used in this experiment are as in table 1; 2) (3-Mercaptopropyl) trimethoxysilane (SH-TMS) was pre-hydrolyzed with HCl (0.01 M) at the H$_2$O/SH-TMS molar ratio of 3:1, used as one of the organosiloxane precursors; 3) 2-[methoxy(polyethyleneoxy)6-9 propyl]trimethoxysilane was pre-hydrolyzed with HCl (0.01 M) at the H$_2$O/PEG-silane molar ratio of 4:1, it was added directly in the pre-condensed phase and used in the goal to modify the external surface of spheroidal organosiloxane submicron/nanoparticles; 4) Triethanolamine was used as condensation catalyst; The SEM image is presented in Figure 7.

**EXAMPLE 19:** Spheroidal organosiloxane submicron/nanoparticles produced with ammonium ions (-N$^+$(CH$_3$)$_3$) grafted on both of internal and external surface). Initial molar composition of the precursors: 21.6 % N-trimethoxysilylpropyl-N,N,N-trimethyl ammonium chloride (TMAPS) as well as 7.2 % C8-TES, 4.0 % PEG$_{6-9}$-TMS and 67.2% TEOS.

Spheroidal organosiloxane sub-micron/nanoparticles were manufactured by following the procedure described in EXAMPLE 2 with the exceptions stated below: 1) The chemicals and the amount used in this experiment are as in table 1; 2) N-trimethoxysilylpropyl-N,N,N-trimethyl ammonium chloride (TMAPS) was pre-hydrolyzed with HCl (0.01 M) at the H$_2$O/TMAPS molar ratio of 3:1, and used as one of the organosiloxane precursors; 3) 2-[methoxy(polyethyleneoxy)6-9 propyl]trimethoxysilane was pre-hydrolyzed with HCl (0.01 M) at the H$_2$O/PEG-silane molar ratio of 4:1, it was added directly in the pre-condensed phase and used with the goal of modifying the spheroidal organosiloxane submicron/nanoparticles with ammonium ions (-N$^+$(CH$_3$)$_3$). The Z-potential of -30 mV was observed as opposed to -50 mV for unmodified spheroidal organosiloxane sub-micron/nanoparticles, which implies the present of ammonium ions (-N$^+$(CH$_3$)$_3$) on the external surface. The SEM image is as in Figure 7.

**EXAMPLE 20:** The spheroidal organosiloxane submicron/nanoparticles produced with ammonium ions (-N$^+$(CH$_3$)$_3$) on only the external surface. Initial molar composition of the precursors: 21.6 % C1-TES 7.2 % C8-TES and 67.2% TEOS.

Spheroidal organosiloxane sub-micron/nanoparticles modified with a positive Z-potential of +8 mV as opposed to unmodified spheroidal organosiloxane sub-micron/nanoparticles with Z-potential of around -50 mV were manufactured by following the procedure described in EXAMPLE 2 with the exceptions stated below: 1) The chemicals and the amount used in this experiment are as in table 1; 2) N-trimethoxysilylpropyl-N,N,N-trimethyl ammonium chloride (TMAPS) was added after 1 hour at the molar ratio of TEOS:TMAPS as 1:9. The flocculation took place after collecting the spheroidal organosiloxane submicron/nanoparticles due to the low zeta potential of +8 mV. The SEM image is presented in Figure 7.

**EXAMPLE 21:** Spheroidal organosiloxane submicron/nanoparticles produced with 100 % bridged silane, 1,2-bis(triethoxysilyl)ethylene (BTEE).

Spheroidal organosiloxane sub-micron/nanoparticles were manufactured with solely organosiloxane precursor of 1,2-bis(triethoxysilyl)ethylene (BTEE) by following the procedure described in EXAMPLE 2 with the exceptions stated below: 1) The chemicals and the amount used in this experiment are as in table 1; 2) 1,2-bis(triethoxysilyl)ethylene was pre-hydrolyzed with HCl (0.01 M) at the H$_2$O/BTEE molar ratio of 2:1 and used solely as the organosiloxane precursors.

The SEM image is presented in Figure 7.

**EXAMPLE 22:** Spheroidal organosiloxane submicron/nanoparticles produced with 100 % TEOS and loaded with BP.

Spheroidal organosiloxane sub-micron/nanoparticles were manufactured with solely organosiloxane precursor of TEOS by following the procedure described in EXAMPLE 2 with the exceptions stated below: 1) The chemicals and the amount used in this experiment are as in table 1; 2) A proportion of 25 wt % of BP defined as the weight ratio (

$$\frac{m_{BP}}{n_{octanoic\ acid} + m_{BP}} \times 100$$

) was solubilised in the octanoic acid oil then added and homogenized with the pre-condensed phase; 3) 2-[methoxy(polyethyleneoxy)6-9 propyl]trimethoxysilane was added directly in the pre-condensed phase and used in the goal to modify the external surface of spheroidal organosiloxane sub-micron/nanoparticles; 3) triethanolamine was used as the condensation catalyst. The final material had an average hydrodynamic particle size of 548 nm and PDI of 0.78. The SEM image is presented in Figure 7.

**EXAMPLE 23:** Spheroidal organosiloxane submicron/nanoparticles produced with one more organosiloxane precursor (large scale). Initial molar composition of the precursors: 18 % C1-TES, 7% C8-TES, 1 % DMDMS and 74% TEOS.

Spheroidal organosiloxane sub-micron/nanoparticles were manufactured by following the procedure described in EXAMPLE 2 with the exceptions stated below: 1) The chemicals and the amount used in this experiment are as in table 1; 2) Dimethyldimethoxysilane (DMDMS) was pre-hydrolyzed with HCl (0.01 M) at the $H_2O$/DMDMS molar ratio of 3:1 and used as one of the organosiloxane precursors; 3) NaOH was used as condensation catalyst. The final material has an average hydrodynamic particle size of 285 nm and PDI of 0.16. The SEM image is presented in Figure 7.

**EXAMPLE 24:** Spheroidal organosiloxane submicron/nanoparticles produced with one more organosiloxane precursor (small scale). Initial molar composition of the precursors: 18 % C1-TES, 7% C8-TES, 1 % SH-TMS and 74% TEOS.

Spheroidal organosiloxane sub-micron/nanoparticles were manufactured by following the procedure described in EXAMPLE 2 with the exceptions stated below: 1) The chemicals and the amount used in this experiment are as in table 1; 2) Small amount of (3-Mercaptopropyl)trimethoxysilane (SH-TMS) added to the prepolymer without hydrolyzing as one of the organosiloxane precursors; 3) TEA was used as condensation catalyst. The final material had an average hydrodynamic particle size of 220 nm and PDI of 0.15. The SEM image is as in Figure 7.

Example 25: Spheroidal organosiloxane submicron/nanoparticles produced with octanoic acid and loaded with BP. Initial molar composition of the precursors: 1% PEG(5000Da)-silane, 21.5% C1-TES, 7.5% C8-TES and 70% TEOS.

Spheroidal organosiloxane nanoparticles were loaded with BP and manufactured with octanoic acid by following the procedure described in EXAMPLE 2 with the exceptions stated below: 1) The chemicals and the amount used in this experiment are as in table 1, 2) A proportion of 20 wt % of BP defined as the weight ratio ( $\frac{m_{BP}}{m_{octanoic\ acid} + m_{BP}} \times 100$ ) was solubilised in the octanoic acid oil then added and homogenized with the pre-condensed phase containing 2-pentanol, 3) The emulsification was accomplished with ultrasonic agitation (50 W, 10 min). The final material had an average hydrodynamic particle size of 90 nm and PDI of 0.2. The SEM image is presented in Figure 7.

Example 26: Spheroidal organosiloxane submicron/nanoparticles produced with octanoic acid and loaded with BP. Initial molar composition of the precursors: 12.5% APTES, 10% C1-TES, 7.5% C8-TES and 70% TEOS.

Spheroidal organosiloxane nanoparticles were loaded with BP and manufactured with octanoic acid by following the procedure described in EXAMPLE 2 with the exceptions stated below: 1) The chemicals and the amount used in this experiment are as in table 1, 2) A proportion of 20 wt % of BP defined as the weight ratio ( $\frac{m_{BP}}{m_{octanoic\ acid} + m_{BP}} \times 100$ ) was solubilised in the octanoic acid oil then added and homogenized with the pre-condensed phase, 3) The emulsification was accomplished with ultrasonic agitation (50 W, 10 min). The final material had an average hydrodynamic particle size of 55 nm and PDI of 0.3. The SEM image is presented in Figure 7.

**EXAMPLE 27:** Spheroidal organosiloxane submicron/nanoparticles produced with oleic acid and loaded with BP. Initial molar composition of the precursors: 22.5% C1-TES, 7.5% C8-TES and 70% TEOS.

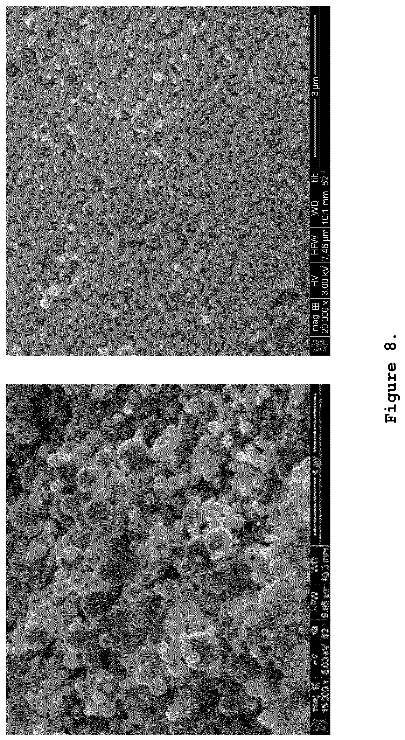

Spheroidal organosiloxane nanoparticles were loaded with BP and manufactured with oleic acid by following the procedure described in EXAMPLE 2 with the exceptions stated below: 1) The chemicals and the amount used in this experiment are as in table 1, 2) A proportion of 25 wt % of BP defined as the weight ratio ( $\frac{m_{BP}}{m_{oleic\ acid} + m_{BP}} \times 100$ ) was solubilised in the oleic acid oil then added and homogenized with the pre-condensed phase. The final material had an average hydrodynamic particle size of 291 nm and PDI of 0.12. The BET surface area, pore volume and pore size were 428 $m^2.g^{-1}$, 0.12 $cm^3.g^{-1}$ and 2.6 nm respectively. The SEM image is presented in Figure 8.

**EXAMPLE 28:** Spheroidal Organosiloxane submicron/nanoparticles produced with 4-methyl-n-octanoic acid and loaded with BP. Initial molar composition of the precursors: 22.5% C1-TES, 7.5% C8-TES and 70% TEOS.

Spheroidal organosiloxane nanoparticles were loaded with BP and manufactured with oleic acid by following the procedure described in EXAMPLE 2 with the exceptions stated below: 1) The chemicals and the amount used in this experiment are as in table 1, 2) 4-methyl-n-octanoic acid was added and homogenized with the pre-condensed phase. 3) TEA was used as condensation catalyst. The final material had an average hydrodynamic particle size of 245 nm and PDI of 0.04. The SEM image is presented in Figure 8.

**EXAMPLE 29:** Negative control experiment: Spheroidal organosiloxane sub-micron/nanoparticles prepared with sodium octanoate instead of octanoic acid. Initial molar composition of the precursors: 22.5% C1-TES, 7.5% C8-TES and 70% TEOS.

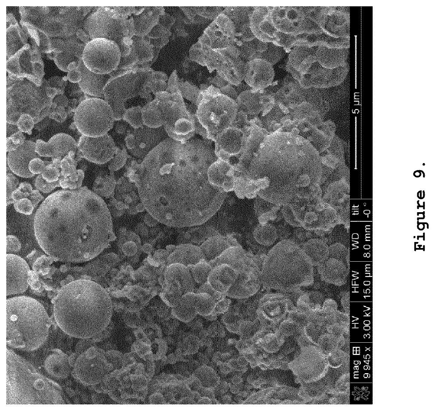

Spheroidal organosiloxane sub-micron/nanoparticles were manufactured by following the procedure described in EXAMPLE 2 with the exceptions stated below. 1) The chemicals and the amount used in this experiment are as in table 1; 2) Sodium octanoate was used instead of octanoic acid. The obtained organosiloxane sub-micron/nanoparticles demonstrated the presence of microspheres organosiloxane (Figure 9), which indicated that octanoic acid plays a crucial role in determining the formation of sub-micron/nanoparticles.

**EXAMPLE 30:** Negative control experiment: spheroidal organosiloxane sub-micron/nanoparticles prepared with caprylic triglyceride instead of octanoic acid. Initial molar composition of the precursors: 22.5% C1-TES, 7.5% C8-TES and 70% TEOS.

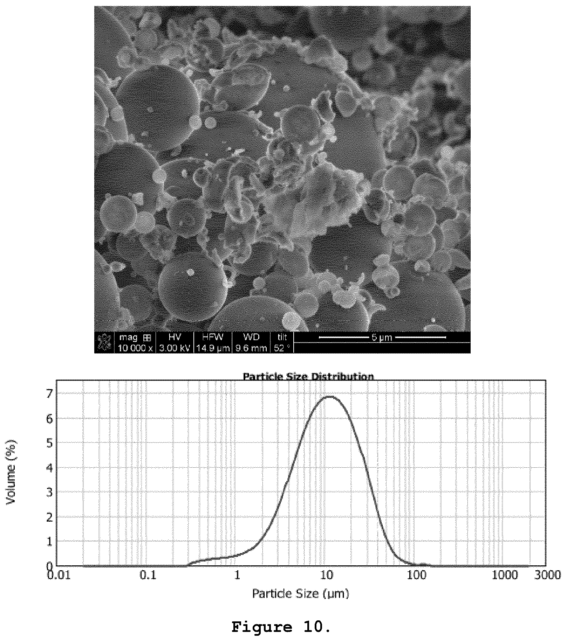

Spheroidal organosiloxane sub-micron/nanoparticles were manufactured by following the procedure described in EXAMPLE 2 with the exceptions stated below. 1) The chemicals and the amount used in this experiment are as in table 1; 2) The volatile solvents in the hydrolyzed organosiloxane precursors were distilled at 100 °C at atmospheric pressure; 3) Caprylic triglyceride was used instead of octanoic acid. The obtained spheroidal organosiloxane sub-micron/nanoparticles demonstrated the presence of microspheres organosiloxane (Figure 10), which indicated that octanoic acid plays a crucial role in determining the formation of spheroidal sub-micron/nanoparticles.

Table 1. The amount of chemicals used in the above examples. The usage of HCl isn't included due to the constant ratio of HCl to corresponding organosiloxane precursors.

| Samples | Organosiloxane precursors | Base /g | Octanoic acid/g | PEG Silane /g | Active/g | $H_2O$ /g |
|---|---|---|---|---|---|---|
| Example 3 | 0.44 g TEOS + 0.12 g C1-TES + 0.06 C8-TES | 2 ml of $NH_4OH$ | 0.33 | - | - | 100 |
| Example 4 | 0.86 g TEOS + 0.24 g C1-TES + 0.12 g C8-TES | 2 mL of $NH_4OH$ | 0.35 | - | 0.075 g of BP | 200 |
| Example 5 | 0.86 g TEOS + 0.24 g C1-TES + 0.12 g C8-TES | 7 ml of TEA | 0.35 | - | 0.075 g of BP | 200 |
| Example 6 | 8.57 g TEOS + 2.36 g C1-TES + 1.22 g C8-TES | 70 ml of TEA | 2.36 | 0.17 ($PEG_{6-9}$-TMS) | 0.78 g of BP | 2000 |
| Example 7 | 0.86 g TEOS + 0.24 g C1-TES + 0.12 g C8-TES | 7 ml of TEA | 0.3 | 0.05 ($PEG_{5K}$-TES) | 0.075 g of BP | 200 |
| Example 8 | 0.74 g TEOS + 0.2 g C1-TES + 0.11 g C8-TES | 2 ml of NaOH (1M) | 0.26 | 0.06 ($PEG_{6-9}$-TMS) | 0.09 g of BP | 180 |
| Example 9 | 0.86 g TEOS + 0.24 g C1-TES + 0.12 g C8-TES | 8 ml of 50 % L-lysine | 0.32 | 0.24 ($PEG_{6-9}$-TMS) | 0.08 g of BP | 200 |
| Example 10 | 2.95 g TEOS + 0.82 g C1-TES + 0.42 g C8-TES | 3.75 ml of $NH_4OH$ | 0.95 | | 0.95 g of a-pinene | 360 |
| EXAMPLE 11 | 0,71 g TEOS + 0.20 g C1-TES + 0.10 g C8-TES | 2 ml of $NH_4OH$ | 0.33 | - | 0.17 g of Vitamin A | 100 |
| EXAMPLE 12 | 0.86 g TEOS + 0.24 g C1-TES + 0.12 g C8-TES | 7 ml of TEA | 0.30 g of Octanoic acid + 0.15 g of triacetin | - | - | 200 |

(continued)

| Samples | Organosiloxane precursors | Base /g | Octanoic acid/g | PEG Silane /g | Active/g | H$_2$O /g |
|---|---|---|---|---|---|---|
| EXAMPLE 13 | 1.04 g TEOS + 0.30 g C1-TES + 0.15 g C8-TES | 7 ml of TEA | 0.91 g of Octanoic acid and 0.45 g DGME | - | 0.05 g of Paclitaxel | 200 |
| EXAMPLE 14 | 0,78 g TEOS + 0.20 g C1-TES | 3 ml of NH$_4$OH (c.c) | 0.25 | - | 0.080 g of BP | 200 |
| EXAMPLE 15 | 1.71 g TEOS + 0.47g C1-TES+ 0.37 g C18-TES | 4 ml of NH$_4$OH (c.c) | 0.47 | 0.056 (PEO 6-9) | 0.16 g of BP | 400 |
| EXAMPLE 16 | 1.04 g TEOS + 0.17g CF3-TMS+ 0.15 g C8-TES | 10 ml of TEA | 0.47 | 0.15 (PEO 6-9) | - | 300 |
| EXAMPLE 17 | 1.04 g TEOS + 0.19g DMDMS + 0.15 g C8-TES | 10 ml of TEA | 0.47 | 0.15 (PEO 6-9) | - | 300 |
| EXAMPLE 18 | 1.04 g TEOS + 0.31g SH-TMS+ 0.15 g C8-TES | 10 ml of TEA | 0.47 | 0.15 (PEO 6-9) | - | 300 |
| EXAMPLE 19 | 1.23 g TEOS + 0.40g TMAPS + 0.175 g C8-TES | 2 ml of NH$_4$OH (c.c) | 0.29 | - | - | 200 |
| EXAMPLE 20 | 0.86 g TEOS + 0.24 g + C1-TES + 0.12 g C8-TES | 7 ml of TEA | 0.30 | - | - | 200 |
| Example 21 | 1.72 g of BTES | 2 ml of NH$_4$OH (c.c) | 0.29 | - | - | 200 |
| Example 22 | 1.7 g of TEOS | 7 ml of TEA | 0.32 | 0.056 (PEO 6-9) | 0.08 g of BP | 200 |
| Example 23 | 0.93 g TEOS + 0.20 g C1-TES + 0.12 g C8-TES + 0.005 g DMDMS | 30 ml of NaOH (1M) | 2.5 | - | - | 200 |
| Example 24 | 0.09 g TEOS + 0.020 g C1-TES + 0.012 g C8-TES + 0.008 g SH-TMS | 0.7 ml of TEA | 0.043 | 0.021 (PEO 6-9) | 0.01 g of BP | 20 |
| Example 25 | 0.2 g TEOS + 0.05 g C1-TES + 0.03 g C8-TES + 0.07 g PEG(5000Da)-silane | 1.5 mL TEA | 0.089 g of octanoic acid and 0.139 g of 2-pentanol | - | 0.02 g of BP | 50 |
| Example 26 | 0.39 g TEOS + 0.06 g C1-TES + 0.04 g C8-TES + 0.74 g APTES | 3 mL TEA | 0.4 | - | 0.1 g of BP | 100 |
| Example 27 | 2.95 g TEOS + 0.82 g C1-TES + 0.42 C8-TES | 3.75 ml of NH$_4$OH | 0.95g of Oleic acid | - | - | 630 |
| Example 28 | 0.86 g TEOS + 0.25 g C1-TES + 0.13 g C8-TES | 7 ml of TEA | 0.3 of 4-methyl-n-octanoic acid | - | - | 200 |
| Example 29 | 0.86 g TEOS + 0.24 g C1-TES + 0.12 g C8-TES | 7 ml of TEA | 0.32 g of sodium octanoate | - | | 200 |

(continued)

| Samples | Organosiloxane precursors | Base /g | Octanoic acid/g | PEG Silane /g | Active/g | $H_2O$ /g |
|---|---|---|---|---|---|---|
| Example 30 | 0.86 g TEOS + 0.24 g C1-TES + 0.12 g C8-TES | 7 ml of TEA | 0.35 Caprilic Triglyceride | - | 0.075 g of BP | 200 |

**Claims**

1. A process of preparation of spheroidal organosiloxane submicron/nanoparticles comprising:

    i1) separately hydrolyzing at least two organosiloxane precursors in a hydrolytic media comprising volatile solvents to provide two or more prehydrolyzed organosiloxane precursors, wherein the two or more silica precursors have the formula $R_{4-x}Si(L)_x$ or formula $(L)_3Si-R'-Si(L)_3$, wherein:

    R: is a mono-valent residue of an alkyl, alkenyl, alkynyl, alicyclic, aryl, alkyl-aryl group, which is optionally substituted by a halogen atom, glycidyloxy-, -OH, -SH, polyethylene glycol (PEG), $-N(R_a)_2$, or $-N^+(R_a)_3$;
    L: is a halogen or an acetoxide $-O-C(O)R_a$, or alkoxide $OR_a$ group;
    R': is a bi-valent residue of an alkyl, alkenyl, alkynyl, alicyclic, aryl, alkyl-aryl group, which is optionally substituted by a halogen atom, -OH, -SH, $-N(R_a)_2$, or $-N^+(R_a)_3$;
    $R_a$ can each independently be hydrogen, alkyl, alkenyl, alkynyl, alicyclic, aryl or alkyl-aryl; and
    X: is an integer of 1 to 4 or alternatively x is an integer of 1 to 3;

    i2) combining the pre-hydrolyzed organosiloxane precursors of step i1) into one container to provide a combined pre-hydrolyzed organosiloxane precursor, and further adding a carboxylic acid-containing compound comprising at least 8 carbon atoms in the the dispersed phase comprising the pre-condensed organosiloxane precursors;
    i3) removing a part or totality of volatile solvents from said combined pre-hydrolyzed organosiloxane precursors to provide a dispersed phase comprising pre-condensed organosiloxane precursors;
    i4) emulsifying, with shear force or sonication, the dispersed phase of the step i3) in an aqueous continuous phase to provide an oil in water nanoemulsion; and
    i5) mixing the nanoemulsion of step i4) with a condensation catalyst to the nanoemulsion of step i4) to obtain the spheroidal organosiloxane submicron/nanoparticles suspension.

2. The process of claim 1, wherein in Step i3), a non-hydrolyzed organosiloxane precursor, other than said two or more organosiloaxe precursors in i1), is added in the dispersed phase.

3. The process of claim 1, step i3), wherein a hydrolyzed organosiloxane precursor, other than said two or more organosiloxane precursors in i1), is added in the dispersed phase.

4. The process of claim 1, wherein in step i3) an active/payload insoluble in the continuous phase is added in the dispersed phase.

5. The process of claim 4, wherein said active/payload insoluble in the continuous phase, is a hydrophobic/liposoluble molecule in a liquid state.

6. The process of claim 4, wherein said active/payload insoluble in the continuous phase, is a hydrophobic molecule in a solid state.

7. The process of claim 4 or 5, wherein said active/payload insoluble in the continuous phase, is a cosmetic or pharmaceutical compound.

8. The process of any one of claims 4 and 5 to 7, wherein said active/payload is a Taxane.

9. The process of claim 1, wherein said carboxylic acid-containing compound is octanoic acid.

Figure 1.

Figure 2.

Figure 3.

PDI for NPs with TEA =0.045

PDI for NPs with NH$_4$OH =0.136

St Dev for NPs with TEA = 35.9 nm

St Dev NPs with NH$_4$OH = 75.7 nm

Figure 3. A) Example 4; B) Example 5

Figure 4.

Figure 5.

Figure 6.

Figure 7.

Figure 8.

Figure 9.

Figure 10.